# EUROPEAN PATENT APPLICATION

(11) **EP 4 811 378 A1**
(43) Date of publication of application: **23.09.2026**
(21) Application number: 26165566.6
(22) Date of filing: 17.03.2026
(51) Int. Cl.: G16H 20/40

(54) **SOURCE-TO-SURFACE DISTANCE ADJUSTMENT AND GRAPHICAL USER INTERFACES**

(30) Priority: 21.03.2025 US 202519087313
(71) Applicant: Varian Medical Systems, Inc., Palo Alto, CA 94304 (US)
(72) Inventor: SPATOLA, Brian, Palo Alto, 94304 (US); VOJAN, Filip, Palo Alto, 94304 (US)
(74) Representative: Mathisen & Macara LLP

(57) **Abstract**

Embodiments disclosed herein relate to adjusting a radiotherapy treatment couch based on real-time Source-to-Skin Distance (SSD) calculations. A display screen 226 on the gantry 900 presents icons 410-424 with setup instructions before treatment. The system calculates the SSD and automatically adjusts the couch to align within a tolerable threshold using a single button instruction. When the correct SSD is reached, a visual indicator confirms proper positioning. For non-zero gantry angles, the system simplifies SSD adjustments by enabling coordinated vertical and lateral couch movements using a single control, reducing operator workload. This approach enhances setup efficiency, reduces cognitive load, and improves treatment accuracy.

## Description

### TECHNICAL FIELD

This application relates generally to graphical user interfaces and software for source-to-surface distance adjustment of radiotherapy treatment setup and machines.

### BACKGROUND

Radiation therapy, which utilizes ionizing radiation, is a localized treatment targeting specific tissues, such as cancerous tumors. Ideally, this therapy focuses on the planning target volume (PTV) or target organ, sparing surrounding healthy tissue from excessive radiation doses to minimize damage. To ensure the prescribed dose is accurately delivered to the PTV, the patient must be precisely positioned relative to the linear accelerator, typically using a movable treatment couch on a turntable assembly. Implementing radiation therapy is a complex process involving specific guidelines, protocols, and instructions followed by various medical professionals, including clinicians, technicians, device manufacturers, and treating physicians. Due to the hazards associated with radiation, it is crucial that all instructions are meticulously followed.

Conventional radiation therapy systems are often complex to operate, requiring extensive training for efficient workflow execution. Tasks such as patient positioning, system calibration, and machine adjustments involve numerous interactions with both the patient and the radiotherapy machine. The data needed for these setups is often voluminous and complex, typically displayed on a screen within the treatment room. However, these conventional methods can be inefficient, ineffective, or even dangerous. Conventional radiotherapy systems do not efficiently display the data needed for patient and machine setup. In some conventional systems and methods, the necessary information for an operator (e.g., radiotherapy technician or any other medical professional) is not presented in a manner that is intuitive and user-friendly, interrupting the adjustment process and proving to be ineffective and time-consuming. Some other conventional systems and methods often provide all the information via static and complex graphical user interfaces (GUIs). Unless an operator is highly experienced with a particular system, this can slow down the setup process and degrade the patient's experience.

### SUMMARY

Radiotherapy encompasses various modalities, including electron therapy, which is a specialized form of external beam radiation that uses high-energy electron beams to treat tumors or areas of concern. In this form of therapy, electrons, accelerated by a linear accelerator, are directed at a target to deliver a precise radiation dose to shallow tissues while sparing deeper structures. This modality is particularly effective for conditions such as skin cancers, post-surgical treatment sites, and keloids, where the tumor or treatment area lies close to the surface. Unlike photon therapies, electron therapy provides sharp dose fall-off beyond the target depth, reducing unnecessary exposure to underlying healthy tissues. As an integral part of the radiotherapy spectrum, electron therapy offers a versatile and safe option for managing specific clinical scenarios, complementing other treatment approaches in achieving optimal patient outcomes. However, electron therapy may require specialized alignment techniques due to its shallow penetration depth and sensitivity to Source-to-Surface Distance (SSD). Therefore, achieving the correct SSD is more important in electron therapy when compared to other treatment modalities. Additionally, electron beams typically require the use of custom applicators or cones for field shaping, further emphasizing the need for meticulous alignment to maintain treatment precision and efficacy.

The concept of adjusting the patient to reach the appropriate SSD in electron therapy involves ensuring the optimal distance between the electron source and the patient's skin to achieve accurate and effective treatment. The SSD is critical for electron therapy because the dose distribution of electrons depends on this distance. An improper SSD can lead to either under-treatment of the target area or over-treatment, affecting both the efficacy and safety of the therapy.

Manual adjustment and patient alignment for SSD adjustments in radiotherapy present significant challenges that can impact the accuracy and efficiency of treatment delivery. The process often requires therapists to iteratively adjust the treatment couch or gantry while visually aligning the patient with projected light fields or other guides. This manual approach is time-consuming and prone to human error, as small miscalculations can lead to incorrect SSD settings, compromising the dose accuracy and potentially harming healthy tissues or underdosing the target area. Variability in operator experience and technique also contributes to inconsistent outcomes. These challenges highlight the need for more automated and intuitive solutions to streamline SSD adjustments, reduce reliance on manual intervention, and ensure precision in patient alignment for radiotherapy treatments.

According to a first aspect of the invention, there is provided a computer-readable medium as defined in claim 1. The computer-readable medium comprises a set of instructions, that when executed, cause at least one processor to present, on a display screen coupled to a radiotherapy machine configured to implement radiotherapy treatment of a patient, a set of icons corresponding to a set of instructions associated with adjusting at least one attribute of the radiotherapy machine before the radiotherapy treatment starts; calculate (e.g. before the radiotherapy treatment starts) a source to skin distance (SSD) for the patient; adjust (e.g. before the radiotherapy treatment starts) a couch and/or a source of the radiotherapy machine, such that the SSD is within a tolerable threshold of a defined SSD; and present (e.g. before the radiotherapy treatment starts), on the display screen, a visual indicator indicating that a correct SSD has been achieved. Corresponding systems and methods are also aspects of the invention. Optional features are specified in the dependent claims. The display screen may be located on a gantry of the radiotherapy machine (or elsewhere). The position of the couch relative to the source of the radiotherapy machine may be adjusted such that the SSD is within a tolerable threshold of a defined SSD (the position of the couch and/or the position of the source may be adjusted). The couch may be a couch for supporting the patient during treatment planning and/or treatment. The source may be an emitter configured to administer radiotherapy to the patient. The source may be an electron source. The defined SSD may be a predefined SSD of a treatment plan for the patient.

According to a second aspect of the invention, there is provided a computer system as defined in claim 5. According to a third aspect of the invention, there is provided a computer system as defined in claim 8. Optional features are specified in the dependent claims.

In one embodiment, the techniques described herein relate to a computer-readable medium including a set of instructions, that when executed, cause at least one processor to: present, on a display screen located on a gantry of a radiotherapy machine configured to implement radiotherapy treatment of a patient, a set of icons corresponding to a set of instructions associated with adjusting at least one attribute of the radiotherapy machine before the radiotherapy treatment starts; calculate a source to skin distance (SSD) for the patient; adjust a couch of the radiotherapy machine, such that the SSD is within a tolerable threshold of a defined SSD; and present, on the display screen, a visual indicator indicating that a correct SSD has been achieved.

The instructions may further cause the at least one processor to: receive an input confirming that the correct SSD is achieved.

The input may be received via an interaction with the display screen or via a pendant of the radiotherapy machine.

The input may be received after aligning a marking on a patient with an indicator emitted on the patient.

The visual indicator may be an additional icon displayed on the display screen or corresponds to a change of a visual attribute for at least one icon within the set of icons.

The instructions may further cause the at least one processor to: configure the at least one attribute of the radiotherapy machine as a default attribute.

The instructions may further cause the at least one processor to: identify an additional adjustment to the at least one attribute; and present the additional adjustment in terms of a default configuration and not an original radiotherapy configuration.

In another embodiment, the techniques described herein relate to a computer system including: a radiotherapy machine having a display screen; and at least one processor in communication with the display screen, the processor configured to: present, on the display screen located on a gantry of the radiotherapy machine configured to implement radiotherapy treatment of a patient, a set of icons corresponding to a set of instructions associated with adjusting at least one attribute of the radiotherapy machine before the radiotherapy treatment starts; calculate a source to skin distance (SSD) for the patient; adjust a couch of the radiotherapy machine, such that the SSD is within a tolerable threshold of a defined SSD; and present, on the display screen, a visual indicator indicating that a correct SSD has been achieved.

The at least one processor may be further configured to: receive an input confirming that the correct SSD is achieved.

The input may be received via an interaction with the display screen or via a pendant of the radiotherapy machine.

The input may be received after aligning a marking on a patient with an indicator emitted on the patient.

The visual indicator may be an additional icon displayed on the display screen or corresponds to a change of a visual attribute for at least one icon within the set of icons.

The at least one processor may be further configured to configure the at least one attribute of the radiotherapy machine as a default attribute.

In yet another embodiment, the techniques described herein relate to a method including: presenting, by at least one processor on a display screen located on a gantry of a radiotherapy machine configured to implement radiotherapy treatment of a patient, a set of icons corresponding to a set of instructions associated with adjusting at least one attribute of the radiotherapy machine before the radiotherapy treatment starts; calculating, by the at least one processor, a source to skin distance (SSD) for the patient; adjusting, by the at least one processor, a couch of the radiotherapy machine, such that the SSD is within a tolerable threshold of a defined SSD; and presenting, by the at least one processor on the display screen, a visual indicator indicating that a correct SSD has been achieved.

A method may further include receiving, by the at least one processor, an input confirming that the correct SSD is achieved.

The input may be received via an interaction with the display screen or via a pendant of the radiotherapy machine.

The input may be received after aligning a marking on a patient with an indicator emitted on the patient.

The visual indicator may be an additional icon displayed on the display screen or corresponds to a change of a visual attribute for at least one icon within the set of icons.

A method may further include: configuring, by the at least one processor, the at least one attribute of the radiotherapy machine as a default attribute.

A method may further include: identifying, by the at least one processor, an additional adjustment to the at least one attribute; and presenting, by the at least one processor, the additional adjustment in terms of a default configuration and not an original radiotherapy configuration.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a workflow-oriented radiotherapy system, according to an embodiment.
**FIG. 2A** illustrates a radiotherapy machine in a default, upright orientation, according to an embodiment.
**FIG. 2B** illustrates a radiotherapy machine in a rotated orientation, according to an embodiment.
**FIG. 3** is a flow diagram of a method for displaying radiotherapy icons on a radiotherapy machine, according to an embodiment.
**FIGS. 4A-D** illustrate a radiotherapy machine displaying a set of icons and instructions, according to an embodiment.
**FIG. 5** illustrates a patient on a couch of a radiotherapy machine, according to an embodiment.
**FIGS. 6A-B** illustrate various cameras and sensors used to achieve the correct SSD and patient alignment, according to an embodiment.
**FIG. 7** illustrates reference markings on a patient, according to an embodiment.
**FIGS. 8A-B** illustrate reference markings on a patient, according to an embodiment.
**FIG. 9** illustrates movement of a couch, according to an embodiment.

### DETAILED DESCRIPTION

Reference will now be made to some embodiments illustrated in the drawings, and specific language will be used here to describe the same. It will nevertheless be understood that no limitation of the scope of the embodiments of the methods and systems described herein is thereby intended. Alterations and further modifications of the features illustrated here, and additional applications of the principles of the embodiments of the methods and systems described herein as illustrated here, which would occur to a person skilled in the relevant art and having possession of this disclosure, are to be considered within the scope of the embodiments, methods, and/or systems described herein.

### System Architecture

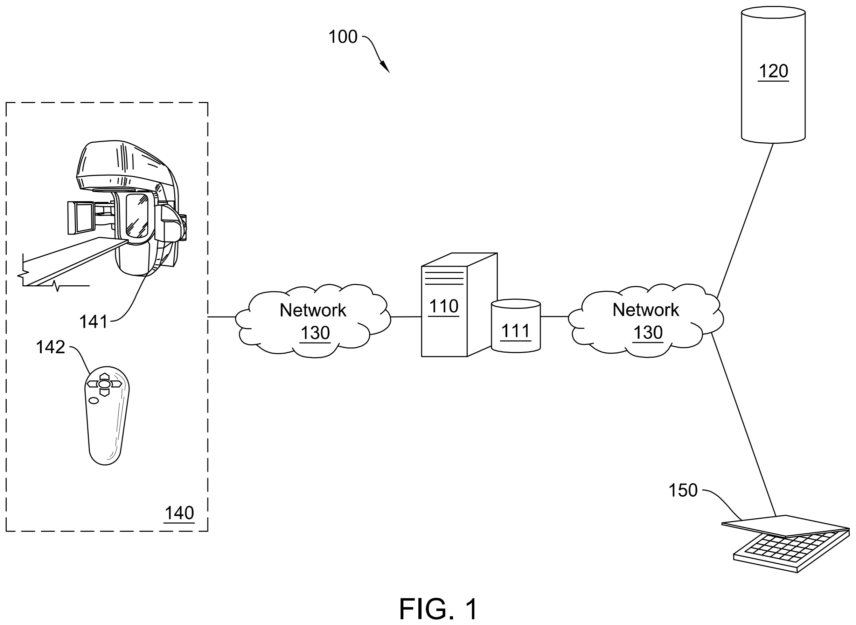

The methods described herein can be implemented using various computing devices/features described in **FIG. 1****.** Therefore, **FIG. 1** describes a non-limiting example of a computer environment where a server can perform the processes/methods described herein, such as retrieving, processing, and presenting radiotherapy treatment data, and presenting data for a graphical user interface (GUI) having workflow-oriented pages (or instances) on various displays screens.

**FIG. 1** illustrates various components of a system **100** for presenting various pages related to operation of a radiotherapy treatment, in accordance with an embodiment. The system 100 may include an analytics server **110**, a medical records database **120**, a radiotherapy system **140**, and a system administrator computer **150** or workstation. These features may communicate with each other over a network **130.** For example, the system **100** may present, via the analytics server **110**, one or more pages/GUIs on the radiotherapy machine **141.** In another example, the system **100** may present, via the analytics server **110**, a page presenting a live feed of a patient on one or more display devices, such as the display device on a gantry of the radiotherapy machine **141.**

The network **130** may include wired and/or wireless communications according to one or more standards via one or more transport mediums. Communication over the network **130** may be in accordance with various communication protocols, such as transmission control protocol and internet protocol (TCP/IP), user datagram protocol (UDP), and Institute of Electrical and Electronics Engineers (IEEE) communication protocols. The network **130** may further include wireless communications according to Bluetooth specification sets, or another standard or proprietary wireless communication protocol. The network **130** may further include communications over a cellular network, including, for example, a global system for mobile (GSM) communications, code division multiple access (CDMA), and enhanced data for global evolution network (EDGE). The examples of the network **130** may include, but are not limited to, private or public local area network (LAN), wireless LAN (WLAN), metropolitan area network (MAN), wide area network (WAN), and the Internet.

The analytics server **110** may be any computing device capable of performing the actions described herein. For instance, the analytics server **110** includes a processing unit and a non-transitory machine-readable storage medium. The processing unit includes a processor with a computer-readable medium, such as a random-access memory coupled to the processor. In some embodiments, the analytics server **110** executes algorithms or computer executable program instructions, which may be executed by a single processor or multiple processors in a distributed configuration. The instructions allow the processor to implement the functionality described herein. The analytics server **110** may be configured to interact with one or more software modules of a same or a different type operating within the system **100.**

Non-limiting examples of the processor may include a microprocessor, an application specific integrated circuit, and a field programmable object array, among others. The analytics server **110** may be capable of executing data processing tasks, data analysis tasks, and valuation tasks. Non-limiting examples of the analytics server **110** may include a desktop computer, a server computer, a laptop computer, a tablet computer, and the like. For simplicity, the **FIG. 1** depicts a single-server computing device functioning as the analytics server **110.** However, some embodiments may include a plurality of server computing devices capable of performing various tasks described herein.

Implementation of the methods described herein is not limited to the system architecture depicted in **FIG. 1****.** In alternative embodiments, the analytics server **110** may be an embedded computing device disposed within the radiotherapy machine **141.** In some embodiments, the analytics server **110** may be a plurality of computing devices operated locally and/or remotely. In various embodiments, the analytics server **110** may be operated through a cloud service (e.g., network, internet). The cloud service may be performed in accordance with various communication protocols such as TCP/IP, UDP, and IEEE communication protocols. The analytics server **110** may utilize a database, such as a local database **111**, to store and/or retrieve various data described herein. For instance, the analytics server **110** may store different data corresponding to the different pages within the local database **111.** The page may be displayed on a display screen associated with the radiotherapy system **140.**

For instance, the analytics server **110** may display a page having a live feed of the patient before the treatment has begun or may display various pages describing how to position the patient on the couch and/or how to adjust the radiotherapy machine **141.** Even though some embodiments describe the analytics server **110** displaying various pages on a display screen attached to the radiotherapy machine **141** (e.g., located on the gantry of the radiotherapy machine **141**), it is expressly understood that the analytics server **110** may display different pages described herein on a display screen located anywhere within the treatment room, such as located on the wall of the treatment room or on any electronic device within the treatment room. In some configurations, the analytics server **110** may display the pages on the system administrator computer **150** or workstation.

The local database **111** may also store data corresponding to the radiotherapy machine **141** (e.g., default position of the radiotherapy machine **141**, current position of the radiotherapy machine **141**, such as the orientation of the bed/couch and gantry).

If the analytics server **110** receives a request from the radiotherapy system **140** to provide the current position/orientation of the radiotherapy machine **141**, the analytics server **110** may query the local database **111** and may be retrieve the corresponding data. Additionally, or alternatively, the analytics server **110** may also communicate with various sensors associated with the radiotherapy system **140** to retrieve machine data and parameters. The analytics server **110** may then use the methods/systems described herein to instruct the radiotherapy machine **141** to adjust the positioning of the radiotherapy machine **141** to the default position of the radiotherapy machine **141** or another position of the radiotherapy machine, or to display one or more pages instructing a technician to adjust the radiotherapy machine **141** and/or the patient.

The local database **111** may also store data associated with different users of the radiotherapy system **140.** In a non-limiting example, the user data may include the user's login (e.g., sign-in, credentials) information and authorization levels. For example, the analytics server **110** may store, using the local database **111** may store, the user's name and password. The analytics server **110** may allow the user to log in to the radiotherapy machine **141**, and/or the system administrator computer **150.** If the user logs-in into the system, the analytics server **110** may adjust what the user is able to view, adjust, and control based on the user's authorization level. The analytics server **110** may conceal the patient's medical information that is not relevant to the radiation treatment from the medical technician. In another example, the analytics server **110** may conceal all of the patient's medical information if a machine technician signs-in.

The analytics server **110** may also utilize one or more other databases, such as the medical records database **120**, to store and/or retrieve various data described herein. The databases herein can be configured as one or more databases storing the data, and the disclosure is not intended to be limited to a particular number or location of databases. The analytics server **110** may instruct the medical records database **120** to store patient data (e.g., patient name, patient machine alignment information) associated with a patient identifier (e.g., patient's name, patient's profile image). The analytics server **110** may then instruct the medical records database **120** to populate a dataset corresponding to a patient and display the profile image of the patient. If the analytics server **110** receives a request from the radiotherapy system **140** to display the data associated with the patient identifier, the analytics server **110** may query the medical records database 120 and may retrieve the corresponding dataset. The analytics server **110** may then use the methods/systems described herein to dynamically display the patient data in accordance with the patient identifier.

The medical records database **120** may also include a radiotherapy treatment file associated with the patient identifier. As used herein, the radiotherapy treatment file refers to all data associated with a patient's radiation therapy treatment and is not limited to a particular step or information. The radiotherapy treatment file may also be retrieved from the radiotherapy system **140**, the medical records database **120**, and/or the system administrator computer **150.** The radiotherapy treatment file may include the treatment data associated with a patient. The radiotherapy treatment file may be associated with a patient identifier and may also be updated after a patient has completed treatment. For example, after the patient has completed a treatment session, the medical records database **120** may retrieve the duration of the treatment session from the radiotherapy system **140** and update the radiotherapy file to include the duration of the treatment session. Even though aspects of the embodiments described herein discuss radiotherapy treatment as a file, the radiotherapy treatment file represents a collection of the patient's medical and treatment data, which may be stored in different files. For brevity, the present disclosure refers to the patient's data as a radiotherapy treatment file.

The radiotherapy treatment file may include data for treatment plans for one or more patients where each treatment plan is specific to a single patient. For instance, each treatment plan is uniquely created for each patient and corresponds to the patient's unique attributes (e.g., physical attributes of the patient and the patient's unique condition to be treated). In some configurations, each treatment plan for each patient may itself include multiple files.

The analytics server **110** may retrieve treatment data associated with a patient that is stored within the patient's radiotherapy treatment file(s). As described herein, the analytics server **110** may analyze the treatment data and may display various features and graphical components described herein. While the medical records database **120** may contain treatment data (radiotherapy treatment files) associated with multiple patients, the methods described herein are implemented such that various pages and graphical features described herein are specific to the particular patient being treated.

In some configurations, the analytics server **110** may retrieve radiotherapy treatment files associated with multiple patients. The analytics server **110** may then receive a selection by an operator (e.g., technician) of a patient to be treated. As a result, the analytics server **110** identifies the radiotherapy treatment file associated with the selected patient and customizes the graphical components described herein for the selected patient.

The analytics server **110** may also retrieve and instruct the medical records database **120** to store patient data associated with the patient from the medical records database **120**, the radiotherapy system **140**, and/or the system administrator computer **150.** For instance, the medical records database **120** may include patient data (e.g., previously populated by the analytics server **110** and/or periodically retrieved from a third-party data source). If a patient is selected, the analytics server **110** may query and retrieve patient data from the medical records database **120** and provide the retrieved patient data. For instance, the analytics server **110** may display the patient's profile image when the patient is selected, providing an opportunity to verify the correct patient was selected.

The analytics server **110** may receive treatment data associated with a patient from the medical records database **120**, the treatment data may further include at least a patient identifier. The analytics server may receive radiotherapy treatment file associated with one or more patients from the medical records database **120.** The radiotherapy treatment file may refer to a file having data associated with a process in which a medical team (e.g., radiation oncologists, radiation therapist, medical physicists, and/or medical dosimetrists) plan the appropriate external beam radiotherapy or internal brachytherapy treatment techniques for a patient.

The data within the radiotherapy file is not limited to the external radiation therapy as other treatments may impact the radiation therapy, such as medical oncology treatment (chemotherapy), interventional oncology treatment (e.g. cryotherapy, microwave therapy, or embolic therapy) or other non-treatment procedures, such as labs and appointments with other medical professionals. The radiotherapy treatment file may include data specific to one or more patient's radiotherapy treatment. The radiotherapy treatment file may include a patient identifier, patient's electronic health data records, medical images (e.g., CT scans, 4D CT scans, MRIs, and x-ray images), treatment-specific data (e.g., arc information or treatment type), target organ (e.g., specification and location data to identify the tumor to be eradicated), treatment plan, etc. Additional examples may include non-target organs, dosage-related calculations (e.g., radiation dose distribution within an anatomical region of the patient), and radiotherapy machine specific information (e.g., couch-gantry orientations, machine trajectory, control points, dose distributions, and/or arc information).

The analytics server **110** may use the patient identifier within the radiotherapy treatment file to identify a particular patient and retrieve additional information regarding said patient. For instance, the analytics server **110** may query the medical records database **120** to identify medical data associated with the patient. For instance, the analytics server may query data associated with the patient's anatomy, such as physical data (e.g., height, weight, and/or body mass index) and/or other health-related data (e.g., blood pressure or other data relevant to the patient receiving radiotherapy treatment). The analytics server **110** may also retrieve data associated with current and/or previous medical treatments received by the patient (e.g., prior treatment fractions, or data associated with the patient's previous surgeries).

The analytics server **110** may analyze the data received and generate additional queries accordingly. For instance, the analytics server **110** may retrieve data associated with one or more medical (or other) devices needed for the patient. The analytics server may retrieve data indicating that the patient suffers from a respiratory medical condition. As a result, the analytics server **110** may generate and transmit a query to the radiotherapy machine **141**, or the system administrator computer **150** to identify whether the patient uses/needs a ventilator.

If necessary, the analytics server **110** may also analyze the patient's medical data records to identify the needed patient attributes. For instance, the analytics server **110** may query a database to identify the patient's BMI. However, because many medical records are not digitalized, the analytics server **110** may not receive the patient's BMI value using simple query techniques. As a result, the analytics server **110** may retrieve the patient's electronic health data and may execute one or more analytical protocols (e.g., natural language processing) to identify the patient's body mass index. In another example, if the analytics server **110** does not receive tumor data (e.g., end-points) the analytics server **110** may execute various image recognition protocols and identify the tumor data.

Another example of a patient attribute may include specific tumor locations. More specifically, this data may indicate the primary tumor location with respect to the patient's centerline. This data may be inputted by the treating oncologist or may be analyzed using various image recognition or segmentation methods executed on the patient's medical images.

Another example of a patient attribute may include whether the patient uses prosthesis (e.g., hip or femoral head prosthesis). This attribute may result in a change of the patient's treatment (e.g., patients with these conditions might require a special treatment).

The analytics server **110** may use various application-programming interfaces (APIs) to communicate with different features described herein. As used herein, an API refers to a computing interface that uses connector programming code to act as a software intermediary between at least two computing components/features described herein. The API may automatically and/or periodically transfer various calls, instructions, and/or requests among different features of the system **100.** Using different APIs, the analytics server **110** may automatically transmit and/or receive calls and instructions.

Additionally, or alternatively, the analytics server **110** may use a content delivery network (CDN) to ensure data integrity when communicating with different features described in the system **100.** As described herein, a CDN refers to a distributed delivery network of proxy servers/nodes that uses multi-layered delivery methods/systems to transmit data (e.g., Akamai). The analytics server **110** may use a CDN when communicating various calls/instructions with the network **130** and/or the local database **111.**

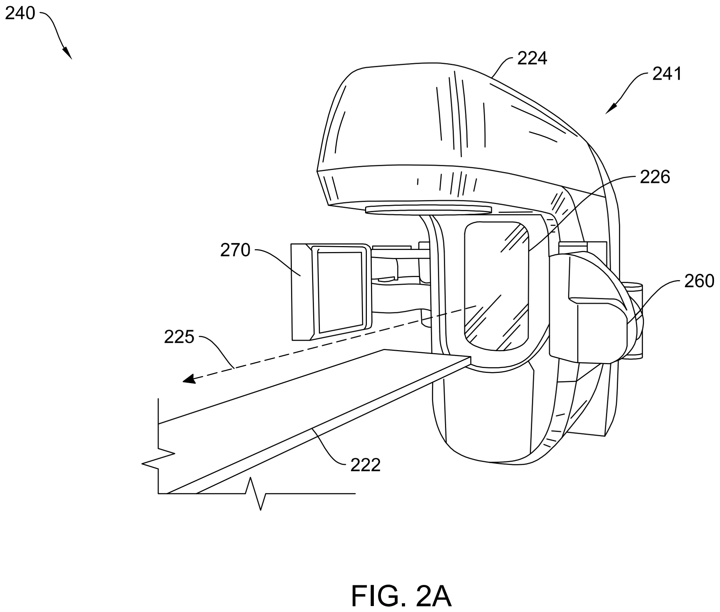

The radiotherapy machine **141** may also include a couch (shown as couch **222** in **FIG. 2A**). to align the patient in a designated position before the treatment begins. The designated position may be identified, calculated, and/or retrieved by the analytics server **110.** For example, the analytics server **110** may retrieve patient data from the medical records database **120**, analyze the data, and utilize the analyzed data to position the patient on the couch. In some embodiments, the doctor identifies how to align the patient on the couch to optimize the treatment therapy. The radiotherapy machine **141** directs radiation at specified locations of the patient resting on the couch during treatment. The specified locations may be selected by the technician operating the radiotherapy machine **141**, the pendant **142**, and/or the system administrator computer **150.** The analytics server **110** may also specify the locations on the patient to direct the radiation.

The radiotherapy machine **141** may also include an x-ray emitter and an x-ray receiver. The x-ray emitter may be disposed on a gantry of the radiotherapy machine **141** and may be configured to provide x-ray (e.g., a penetrating form of high-energy electromagnetic radiation) waves. The x-ray emitter emits x-ray waves to the patient who is positioned on the couch. The x-ray receiver may be disposed opposed to the x-ray emitter. The x-ray waves received by the x-ray receiver generate an imprint (e.g., image) of the patient's internal anatomy. Although the example embodiment recites the use of x-ray imaging, an alternative configuration for the radiotherapy machine may include an additional or other medical imaging apparatus (e.g., fluoroscopy apparatus, MRI, etc.).

The radiotherapy machine **141** may also include a set of cameras (e.g., camera on an end of the couch, gantry camera, ceiling camera, or other cameras placed within the radiotherapy room). The analytics server **110** may retrieve a live feed of the couch (e.g., with or without the patient) from the set of cameras and provide it to a display screen disposed on the radiotherapy machine **141.** The set of cameras may also directly communicate with the radiotherapy system **140.**

The radiotherapy machine **141** may also include a gantry that may be in communication with the analytics server **110.** If the radiotherapy machine **141** is in operation, the gantry may rotate relative to the radiotherapy machine **141** to begin the treatment of the patient. The analytics server **110** may use the live feed of the set of cameras to control and/or stop the movement of the gantry. During operation, the analytics server **110** may instruct the display screen of the radiotherapy machine **141** to display the live feed of the set of cameras and/or a designated page to the display screen of the radiotherapy machine **141.** In various embodiments, the analytics server **110** may not instruct the display screen of the radiotherapy machine **141** to activate the display screen of the radiotherapy machine **141** during operation. In some embodiments, the display screen of the radiotherapy machine **141** may be a touch screen and may control the pages generated by the analytics server **110** through the display screen of the radiotherapy machine **141.**

The radiotherapy machine **141** may have a default home position. Before the patient receives treatment, the radiotherapy machine **141** may be reset to a position that is ergonomically desirable for the patient. The default home position may also be optimized to receive a new patient or allow the patient to more easily get off the couch.

As discussed in greater detail herein, the radiotherapy machine **141** may also be controlled by the pendant **142.** The pendant **142** may be connected to the radiotherapy machine **141** through wired or wireless communications according to, for example, Bluetooth specification sets or another standard or proprietary wireless communication protocol. In various embodiments, the pendant **142** may be connected to the radiotherapy machine **141** through a wired connection or be integrated into the radiotherapy machine **141.** The pendant **142** may also be in communication with the analytics server **110.** The pendant **142** may adjust the positioning of the radiotherapy machine **141** through a selection of buttons that axially actuates the radiotherapy machine **141** (e.g., couch) towards a desired direction. The technician may also use the pendant **142** to initialize the radiotherapy machine **141**, and/or to have the radiotherapy machine **141** return to its home position.

The pendant **142** may also allow the technician to input patient information, which may be then communicated to the radiotherapy machine **141**, the analytics server **110**, and/or the medical records database **120.** The pendant **142** may be used to control one or more of the steps of the setup and/or treatment of the patient.

The pendant **142** may also include a touch pad (e.g., tactile sensor). The touch pad may be used to control various pages generated by the analytics server **110.** For example, the user may use the touch pad of the pendant **142** to control a page, generated by the analytics server **110**, to proceed through the workflow steps of the radiotherapy setup and/or treatment. In some embodiments, the page may be controlled through a touch screen on the radiotherapy machine **141** or elsewhere in the treatment room.

As discussed in greater detail herein, the radiotherapy system **140** may further include accessories that assist in the radiotherapy setup and/or treatment of the patient. The analytics server **110** may communicate with the radiotherapy system **140** to select which accessories are required. The analytics server **110** may also communicate with the medical records database **120**, and/or the system administrator computer **150** to determine which accessories are required. For example, a patient may require a specific accessory for their radiotherapy setup and/or treatment. The analytics server **110** may retrieve that the patient requires a specific accessory by accessing the medical records database **120** and communicate that information to the radiotherapy system **140.** The accessories may in communication with the radiotherapy system **140.** For instance, as will be described below, the analytics server may use RFID tags to scan and identify the location and/or presence of various accessories. In some embodiments, the accessories are wired or otherwise integrated into the radiotherapy machine **141.**

The local database **111** associated with the analytics server **110**, the medical records database **120**, and the radiotherapy machine **141** are capable of storing information in various formats and/or encrypted versions. The information may include data records associated with various patient information, user preferences, a set of prompts (e.g., question, query, and inquiry), attributes associated with various pages to be generated by the analytics server **110**, and the like. The medical records database **120**, may have a logical construct of data files, which are stored in non-transitory machine-readable storage media, such as a hard disk or memory, controlled by software modules of a database program (e.g., structured query language (SQL)), and a database management system that executes the code modules (e.g., SQL scripts) for various data queries and management functions.

The system administrator computer **150** may represent a computing device operated by a system administrator. The system administrator computer **150** may communicate with the analytics server **110.** The system administrator computer **150** may be configured to display various analytic metrics where the system administrator can monitor gantry movement, patient information, and modify various thresholds/rules described herein. The system administrator computer **150** may be configurable to display certain analytics metrics when specific thresholds/rules have been exceeded. For example, the system administrator computer **150** may be alerted if a patient has moved a specified amount during treatment. The analytics server **110** may allow the system administrator computer **150** to also control and/or override the settings of the radiotherapy machine **141**, and/or the pendant **142.** For instance, an administrator may revise the minimum distance threshold and/or customize any feature on the pages described herein (e.g., move features within the page, color, font, shape, size, or the order of display for one or more pages).

The analytics server **110** may configure the system administrator computer **150** to review any and/or all commands made through the radiotherapy system **140** at specified process steps. The system administrator computer **150** may then allow or reject the commands made through the radiotherapy system **140.** For example, before the technician starts the patient treatment using the radiotherapy machine **141**, the analytics server **110** may first prompt the system administrator computer **150** to review the radiotherapy machine **141** parameters for approval. Once approved, the technician may then control the radiotherapy machine **141.**

The analytics server **110** may configure the system administrator computer **150** to review any and/or all reading and/or (over)writing of patient data to and/or from the medical records database **120.** For example, before the technician may (over)write patient information collected by the radiotherapy machine **141**, the analytics server **110** may first prompt the system administrator computer **150** to review the patient information before it is stored in the medical records database **120.**

**FIG. 2A** illustrates, as described above, a radiotherapy system **240** including a radiotherapy machine **241** that includes a gantry **224** that rotates about a center axis **225** of the radiotherapy machine **241.** In some embodiments, the radiotherapy system **240** may be substantially similar to the radiotherapy system **140** of **FIG. 1****.** Likewise, the radiotherapy machine **241** may be substantially similar to the radiotherapy machine **141** of **FIG. 1****.** The radiotherapy machine **241** may further include a display screen **226** positioned on or within the gantry **224.** The display screen **226** may be configured to display for view to a user (e.g., a technician, patient, and/or a medical practitioner) relevant information related to the radiotherapy procedure before, during, and/or after the radiotherapy procedure.

The radiotherapy machine **241** may also include an imager **270** and an emitter **260** for administering the radiotherapy to a patient. The radiotherapy system **240** may additionally include a couch **222**, upon which the patient may be placed before and/or during the administration of the radiotherapy (e.g. for imaging by imager 270 prior to radiotherapy treatment). In some embodiments, the couch **222** may be translated laterally relative to the radiotherapy machine **241** (e.g., along the center axis **225**) and/or rotated axially relative to the center axis **225** of the radiotherapy machine **241.** Likewise, in some embodiments, the radiotherapy machine **241** may be rotated axially about the center axis **225**, and relative to the couch **222**, before, during, and/or after the administration of the radiotherapy treatment.

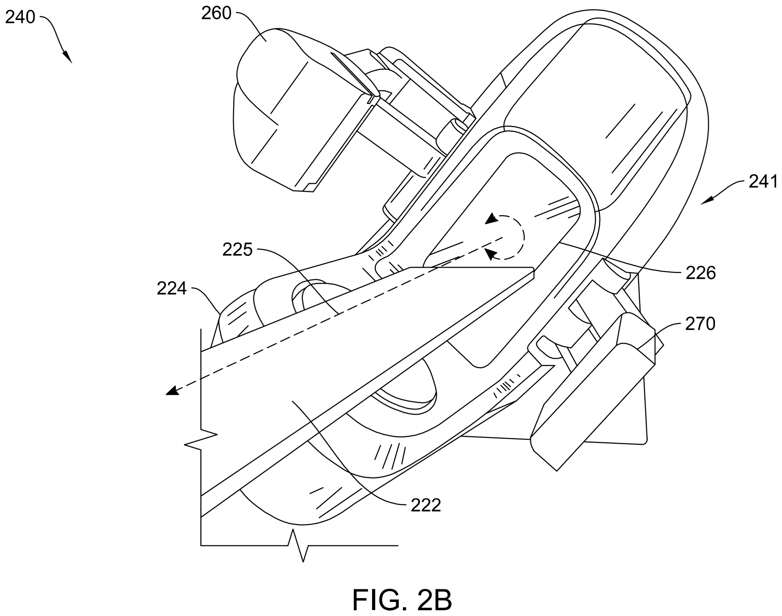

For example, as illustrated in **FIG. 2B**, the radiotherapy machine **241** is shown in a rotated position about the center axis **225.** Relative to the default position of the radiotherapy machine **241** (as shown in **FIG. 2A**), the radiotherapy machine **241** in **FIG. 2B** is shown at a position that is rotated about 135.5° counter-clockwise. Because the display screen **226** is coupled to the gantry **224** of the radiotherapy machine **241**, the display screen **226** is also rotated from a default, upright position (as shown in **FIG. 2A**) at about 135.5° counterclockwise.

Using the methods and systems discussed herein, one or more processors can automatically adjust the radiotherapy machine to achieve the correct Source-to-Surface Distance (SSD). The concept of adjusting the SSD may involve modifying the distance between the radiation source, typically housed within the linear accelerator or gantry or refined by an accessory or an applicator, and the surface of the patient or treatment area. The SSD may affect the intensity and focus of the radiation beam, making accurate settings essential for aligning with the treatment plan.

The methods and systems discussed herein allow for optimizing the alignment and configuration of radiotherapy machines (e.g. alignment and configuration of radiotherapy machines prior to treatment by the radiotherapy treatment machine), particularly for procedures such as electron therapy, where precise SSD adjustments are critical. By leveraging real-time input from sensors, cameras, or surface mapping technologies, the methods and systems discussed herein can dynamically update positional parameters and guide the operator in achieving the correct SSD. Additionally, the methods and systems discussed herein provide intuitive visual cues on the display screen to indicate that the correct SSD is achieved.

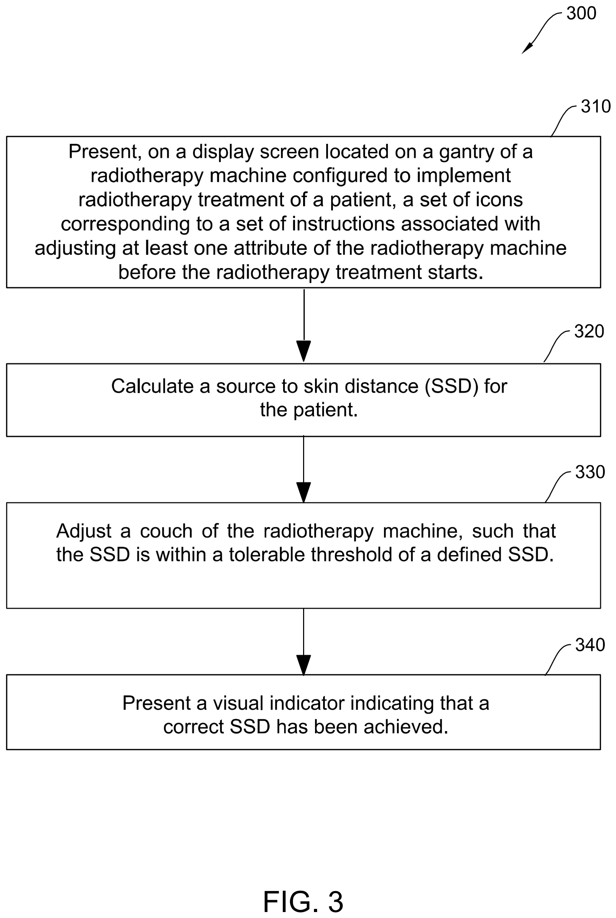

**FIG. 3** is a flowchart of an example method **300** for displaying one or more icons and instructions on a display screen coupled to a radiotherapy machine. The method **300** may be executed by one or more processors described herein, such as the analytics server **110**, the pendant **142**, the system administrator computer **150**, the radiotherapy system **140**, the radiotherapy machine **141**, etc. The one or more processors may execute instructions stored on a computer-readable, non-transitory medium, which may cause the one or more processors to execute steps of the method **300.** While the steps of the method **300** are shown in a specific order, it should be understood that the method **300** may comprise more, fewer, and/or different steps than those depicted in **FIG. 3****.** Likewise, the order of the steps of the method **300** is shown in **FIG. 3** for illustrative purposes. However, it is understood that the steps of method **300** may be performed in any number of configurations or orders without departing from the scope of the systems and methods described herein.

At step **310**, one or more processors may present, on a display screen located on a gantry of a radiotherapy machine configured to implement radiotherapy treatment of a patient, a set of icons each corresponding to a set of instructions associated with the radiotherapy treatment and/or setup.

As discussed herein, one or more processors may generate a set of tasks for configuring a radiotherapy treatment and/or setup for a patient. Each task within the set may be associated with a specific stage of the radiotherapy treatment and/or setup, facilitating a structured and workflow-oriented approach to treatment planning and/or execution. In some embodiments, the one or more processors may first retrieve a radiotherapy file (RT file) associated with the patient in order to identify the patient's treatment data.

As used herein, the RT file may refer to a comprehensive collection of data associated with a patient's radiotherapy treatment, encompassing all information necessary for setup, planning, executing, and//or monitoring the treatment process. In some embodiments, the RT file may include patient identifiers, such as names and demographic details, along with imaging data like CT scans, 4D CT scans, MRIs, and x-rays that provide anatomical details for treatment planning. The RT file may also contain detailed treatment plans, including beam trajectories, dose levels, arc information, and the type of radiotherapy being administered. Additionally, the RT file may specify target and non-target organ data to ensure accurate radiation targeting while minimizing exposure to healthy tissues.

Additionally, the RT file may include patient-specific attributes such as physical characteristics (e.g., height, weight, BMI) and medical history, as well as details about required accessories like immobilization devices or bolus materials. Session-specific data, such as couch positioning and adjustments from prior treatments, are also recorded to enable consistency across treatment sessions. The RT file may serve as a resource for the one or more processors, enabling the dynamic generation of workflow tasks (e.g., the values displayed for each icon), real-time updates to the GUI, and personalized treatment workflows. This structured approach allows the GUI to be populated with precise and efficient radiotherapy tailored to the unique needs of each patient. By leveraging the patient's RT file, medical records, and treatment data, the one or more processors may identify and define tasks tailored to the patient's unique attributes and treatment requirements.

The set of tasks may correspond to various stages of the radiotherapy treatment and/or setup, such as patient alignment (before and/or during treatment), machine calibration (before and/or during treatment), accessory verification (before and/or during treatment), dose administration, post-treatment analysis, and/or the like. For instance, during the alignment stage (e.g. during treatment setup), a task may involve verifying the patient's position on the treatment couch relative to the isocenter of the radiotherapy machine. The one or more processors may generate tasks to guide the operator in adjusting the couch and gantry to achieve the required alignment.

After the tasks and icons have been identified, the one or more processors may display the icons. In some embodiments, the display screen, e.g integrated into the gantry, may provide a visually accessible/interactive interface that assists operators in efficiently navigating through treatment and/or setup tasks without diverting attention from the patient or the machine. The GUI may display various icons where each icon corresponds to a specific task within the treatment and/or setup workflow, such as patient alignment (before and/or during treatment), machine calibration (before and/or during treatment), or dose administration, and provides intuitive visual indicators to guide the operator through the sequential stages of the treatment and/or setup process.

The set of icons may be radially arranged on the display screen to enhance accessibility and usability during operation. In this way, the icons (or at least a portion of the icons) can be displayed simultaneously. The radial configuration allows operators to easily identify and interact with individual icons regardless of the screen's orientation or the gantry's position. The radial arrangement is designed to optimize spatial organization and ensure that icons remain legible and logically grouped even as the gantry rotates. In some embodiments, the one or more processors may dynamically update the icons based on task completion, visually distinguishing completed tasks and highlighting the next actionable steps in the workflow. This arrangement, combined with real-time updates, improves operational efficiency by reducing cognitive load and enabling seamless progression through the radiotherapy treatment and/or setup process.

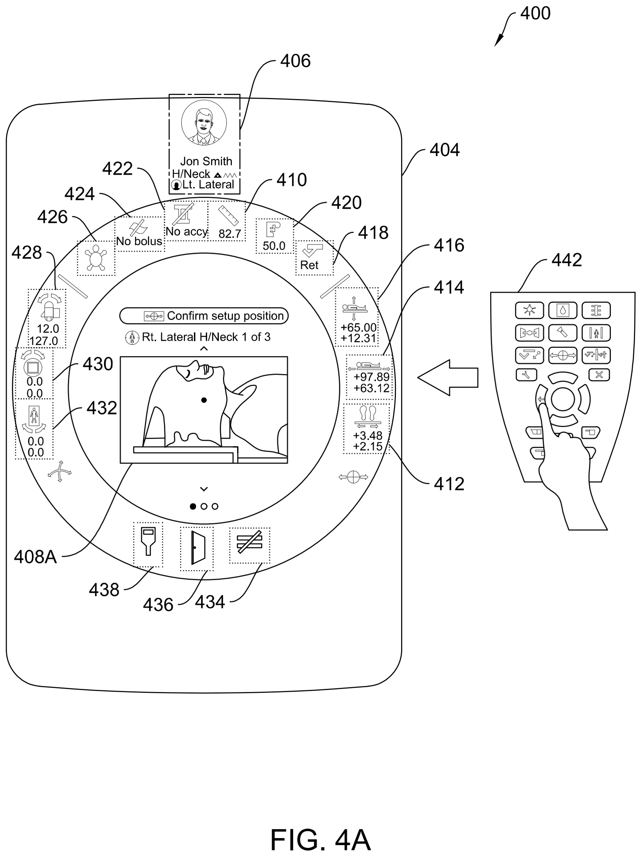

The radial arrangement may place the icons around a center point of the display screen (e.g., center portion **408** discussed in **FIGS. 4A-B**), allowing the icons to be uniformly distributed across the display screen.

In some embodiments, the icons may be displayed radially with each icon positioned at an equal radius from the central point on the display screen. This uniform radial distance may provide a balanced icon layout, where each icon is equidistant from the center point of the display screen, making them easily accessible and visually consistent. Such a configuration minimizes cognitive effort for the operator, as icons are uniformly-spaced and their positions can be intuitively located within the circular arrangement.

The icons may form a full circle or a semi-circle around the central point, depending on the screen size, radiotherapy machine type, the number of icons, and the workflow requirements. In some embodiments, a full circle arrangement may be used for maximizing icon capacity while maintaining symmetry (e.g., when multiple tasks need to be displayed simultaneously). In contrast, a semi-circle arrangement can be used to emphasize a subset of tasks or to make space for additional interface elements, such as dynamic instruction displays or task-specific information. These configurations can adapt dynamically to the operator's needs, presenting icons in a visually organized manner while keeping all relevant information within the same page.

In embodiments where a full-circle arrangement is used, the central point of the display screen could be reserved for detailed task instructions, with the icons evenly distributed around it. In a semi-circle arrangement, the bottom half of the screen could be left open for visual indicators, such as task progress bars or alerts, while the upper semi-circle (and sometimes side portions) may contain the workflow icons. The radial design, whether a full or partial circle, ensures that the icons remain logically grouped and easy to access, even as the gantry or display rotates, maintaining operator efficiency and usability.

The layout of the radial display may allow the operator to easily locate and interact with each icon regardless of the gantry's rotation or the display's orientation. By organizing the icons radially, the system minimizes visual clutter while maintaining a logical grouping of the icons.

The icons displayed on the display screen may be grouped based on predefined attributes such as function, workflow stage, or type of information, and these groupings can be customized or modified to suit the preferences of different operators or clinics. For instance, icons related to patient information, such as demographic data or treatment specifics, may be grouped separately from icons representing machine setup tasks, such as couch alignment or gantry positioning. The predefined grouping criteria may allow for consistency across workflows while allowing for flexibility to adapt to varying clinical protocols or individual operator needs. Customization options enable clinics to tailor the grouping and presentation of icons based on their unique workflows, equipment configurations, or operational preferences. This adaptability can be used to ensure that the system remains intuitive and efficient, regardless of the user's specific requirements or the treatment environment.

In some embodiments, the icons may be arranged in a sequential order that reflects the typical (e.g., pre-defined) workflow of the treatment and/or setup process (before and/or during treatment), ensuring tasks are performed in the correct sequence and guiding the operator intuitively through each stage. As a result, the location of the icons may correspond to the order of the tasks. For instance, the icons may be arranged such that a subsequently displayed icon (when considering the display of the icons clockwise or counterclockwise depending on the setup of the icons) may correspond to the next task to be accomplished.

In a non-limiting example, the icon arrangement starts with a patient identification icon, placed at the beginning (most left or most right of the displayed icons) to allow the operator to verify the patient's identity and treatment plan, ensuring the correct individual is being treated or will be treated in the future. This foundational step minimizes the risk of errors and sets the stage for subsequent tasks. Following this, all other icons may be arranged in a clockwise manner, such that the operator can intuitively understand which task must be performed next.

This logical and workflow-oriented arrangement of icons ensures that operators are intuitively guided through the radiotherapy and/or setup process (before and/or during treatment), reducing cognitive load and minimizing errors. The sequence of icons can also be dynamically adjusted by the analytics server based on real-time task completion or user inputs, providing flexibility to accommodate specific scenarios or operator preferences. This approach ensures that the GUI remains efficient, user-friendly, and aligned with the needs of the radiotherapy and/or setup workflow.

In addition to corresponding to a specific task, each icon may also be dynamically interactive, adapting to changes in the treatment environment and user inputs. This dynamic adaptation may be driven by the analytics server (or any other server/processors discussed herein), which monitors the progress of each task and transitions the GUI to display/highlight the next set of icons upon task completion. For instance, once the alignment of the patient is verified, the corresponding icon may be updated (e.g., visually by changing colors) to indicate completion, while the next icon highlights the verification of dose calibration. This sequential and responsive design ensures that the operator is guided seamlessly through the treatment workflow and/or setup (before and/or during treatment) without needing to be directed to a new page and while the viewing status of other icons/tasks, enhancing efficiency and reducing setup time. Moreover, the distinct visual and functional characteristics of each icon-such as color changes, animations, or text overlays-serve as intuitive indicators of task status, enabling quick recognition and action by the operator.

Referring now to **FIG. 4A**, a display of the icons on a display screen of a radiotherapy machine is presented, in accordance with one embodiment. The set of icons, in this embodiment **400A** are presented on a display screen **404** (similar to the display screen **226**) located on a gantry of a radiotherapy machine. As depicted, the icons **406-432** are displayed radially on the display screen **404**, though in other embodiments, the arrangement may be different. The display screen may also include a center portion **408A-C** that can display additional data needed or otherwise associated with different icons.

As depicted, the set of displayed icons includes an icon **406** that may include the patient's personally identifiable information, such as the patient's image, name, diagnoses, and other information that can identify the patient.

Different groupings of icons (or sometimes individual icons) may be arranged in accordance with a predefined order that may or may not correspond to the workflow of the patient's radiotherapy treatment and/or setup. For instance, in one embodiment, different tasks to be performed may be associated with a defined order. As a result, their corresponding icons may be arranged in that order.

In other embodiments, such as the depicted embodiment, the icons may be arranged in accordance with a grouping of the tasks. For instance, icons may be arranged based on whether they are associated with a patient setup category, radiotherapy accessory category, radiotherapy machine attribute category, rotational parameter category, interlocks, or a system status category. As depicted, the set of icons may be divided into three groups where the groups indicate a cluster of tasks to be performed. The cluster of tasks may have a common attribute and may be clustered based on the common attribute. For instance, one grouping or cluster of icons may indicate adjustments to be made to the patient (e.g., patient alignment) and another grouping of the icons may be directed to machine adjustment.

A first grouping of the icons may include patient alignment indicators (e.g., icons **412**, **414**, and **416**)**.** The icon **412** may indicate whether the patient needs to be laterally moved (e.g., moved to the left or right). Additionally, the set of icons may include an icon **414** that indicates whether the patient should be moved on the couch (e.g., toward the radiotherapy machine or away from the machine). Additionally, the set of icons may include an icon **416** that indicates whether the patient on the couch should be moved vertically (e.g., whether the couch should be moved up or down).

A second grouping of icons may include machine/treatment accessories and may include icons **410**, **418**, **420**, **422**, and **424.** The icon **410** may correspond to a distance indicator measuring/indicating the distance between the radiation source and the patient's skin (source-to-skin distance, SSD). Additionally, the set of icons may include an icon **418** that indicates whether an imager of the machine is extended. Additionally, the set of icons may include an icon **420** that indicates the status of the imagers of the machine (e.g., how far the imagers are from the machine). Additionally, the set of icons may include an icon **422** that indicates whether an accessory (and if so, which accessory) is needed to image and/or treat the patient. Additionally, the set of icons may include an icon **424** that indicates whether a bolus is needed to treat the patient. Additionally, the set of icons may include an icon **426** that indicates the patient orientation (e.g., whether the patient should be on their back with their head toward the machine or otherwise). Additionally, the set of icons may include an icon **428** that indicates whether any adjustment to the gantry is needed. The icon **428** may include arrows indicating which direction the gantry should be turned or otherwise moved towards. In some embodiments, the icon **428** may also include an angle value indicating a desired position of the gantry.

Another grouping of the icons may include machine attribute icons that correspond to tasks related to adjusting the radiotherapy machine. This group may include icons **428**, **430**, and **432.** The icon **430** may indicate whether any adjustments are needed to be made to the collimator. In some embodiments, the icon **430** may include a map of the collimator opening. Additionally, the set of icons may include an icon **432.**

In some embodiments, system administrators or operators may have the capability to adjust any attribute of the icons displayed on the display screen, including visual attributes such as color, font style, size, line thickness, shading, and/or the like. This flexibility allows the icons to be modified to align with institutional branding, operator preferences, or accessibility requirements. For instance, line thickness or font size can be increased to enhance visibility for users with visual impairments. These adjustments ensure that the interface remains both functional and user-friendly, tailored to meet the specific needs of the clinical setting.

The position of the icons on the display screen can also be adjusted by system administrators or operators to optimize usability and align with operator workflows. Icons can be repositioned within the radial arrangement or moved to alternative layouts, ensuring that the most frequently accessed icons are conveniently located. This customization enables the interface to adapt to specific clinic requirements or individual user preferences, improving efficiency during radiotherapy procedures and/or radiotherapy setup procedures. For instance, a clinic focusing on particular types of treatments may prioritize the placement of certain icons for quick access, minimizing navigation time and enhancing operational precision.

In some embodiments, a system administrator or an operator can be authorized to add new ones to better reflect the workflow or accommodate updates to the radiotherapy process. This capability ensures that the interface remains relevant and streamlined, reducing visual clutter and focusing only on the tasks and information pertinent to the specific clinical scenario. For example, an icon can be added to the set of icons that indicates whether a door of the treatment room is open, whether a predicted collision is going to occur, whether the pendant is properly connected to the radiotherapy machine, and/or the like.

The set of icons may also include an icon **438** indicating whether a pendant is properly connected to the radiotherapy machine, icon **438** indicating whether the treatment room door has been properly closed, and/or icon **434** indicating whether the machine parameters are or are not in the planned positions.

The icons displayed may be interactive, such that the operator can move along the icons and select (e.g., click, press, touch, or otherwise interact with different icons), e.g., using a pendant of the radiotherapy machine, such as the pendant **442.** For instance, the operator may use one or more buttons on the pendant **442** to navigate through the displayed icons and use another button to make a selection.

In some embodiments, the one or more processors may present a visual indicator on the display screen indicating that the subset of the set of instructions satisfies a threshold, and the subset of the set of instructions is displayed in a plurality of pages. The visual indicator may be designed to inform the operator that a subset of the set of instructions satisfies a predefined threshold. As used herein, the threshold may correspond to any attribute that is defined by a system administrator or an operator. For instance, the threshold may correspond to a number of instructions for the selected icon. In another example, the threshold may indicate a predefined progression of instructions that are designed to be separately and/or consecutively displayed. For example, when the subset of instructions exceeds the display capacity of a single page, the one or more processors organize and display the instructions across a plurality of pages, ensuring that all relevant information is accessible without overwhelming the interface or compromising usability.

In some embodiments where the subset of instructions contains numerous detailed steps, such as alignment adjustments, gantry configuration, or dose calibration, the one or more processors may distribute these steps across multiple pages. Moreover, the one or more processors may display a visual indicator informing the operator that the instructions are displayed on multiple pages. Non-limiting examples of a visual indicator may include visual navigation cues, such as pagination icons, scrolling indicators, toggle icons, or a progress bar, and the like. The visual indicator may allow the operator to seamlessly navigate between pages while ensuring clarity and task focus. For instance, the indicator may convey the number of pages and the position of the current page (among the set of pages) being displayed. This functionality ensures that the interface remains efficient and user-friendly, even when managing complex or large sets of instructions, ultimately improving workflow efficiency and treatment accuracy.

For instance, and still referring to **FIG. 4A**, the one or more processors may determine that the selected icon includes more information than can be displayed on one page. As a result, the one or more processors may display the visual indicator **440A** indicating that the information displayed in the center portion **408A** includes multiple pages. The visual indicator **440A** indicates that there are three total pages (e.g., three dots) and further indicates that the currently displayed page is the first of three pages.

Referring back to **FIG. 3**, as step **320**, the one or more processors may calculate an SSD (or a current or actual SSD) for the patient. The SSD may be calculated/determined prior to treatment. The one or more processors may use a variety of methods to determine the actual or current SSD between the patient's skin (of the target area) and the source (e.g., the defined section of the gantry that emits the electrons for treatment) as the patient is located on the couch.

First, the one or more processors may determine that the planned treatment is electron therapy. The one or more processors may determine that the patient is undergoing electron therapy or has electron therapy planned, as adjusting SSD might only be performed for patients undergoing this modality of therapy or having it planned. The one or more processors may make this determination using a variety of methods. For instance, in one example, the RT file, that includes treatment attributes, may indicate that the patient is receiving electron therapy or has it planned. In another example, the operator may manually interact with an icon and/or a button on the pendant (or any other input element, such as a console computer) to indicate that the patient is receiving electron therapy or has it planned.

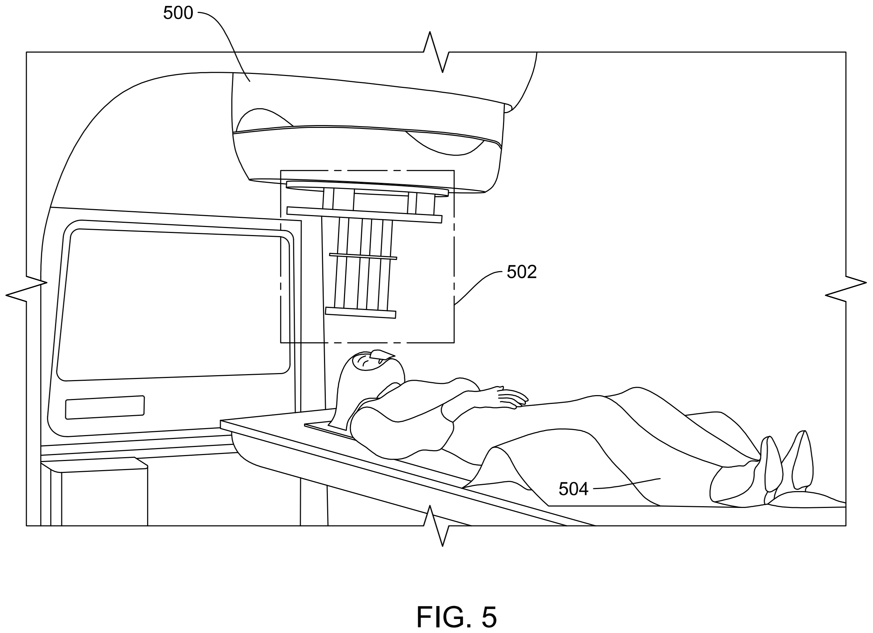

Electron therapy patient setup is different than other radiotherapy setups because, in electron therapy setups, the machine uses specialized attachments, like cones or applicators, to shape the electron beam for precise targeting. Referring now to **FIG. 5**, a non-limiting example of an electron setup is provided. As depicted, the radiotherapy machine **500** may include an applicator 502 that ensures that the electrons are directed toward the target area of the patient **504.**

The applicator **502** ensures that the treatment area receives the intended dose while sparing adjacent healthy tissues. In some embodiments, the target area is marked before the treatment has commenced. After which a covering (e.g., lead covering) is placed on the patient's target area ensuring that the rest of the patient's body does not receive any harmful effects.

Referring back to step **320** of **FIG. 3**, at step **320**, the one or more processors may determine the SSD using one or more manual inputs, sensors, imaging technologies, and/or machine parameters that measure and calculate the precise distance between the radiation source and the patient's skin or treatment surface.

In one example, the SSD may be manually measured and entered by the operator. For instance, the operator may manually measure (sometimes a light may be shined onto the patient where the light indicates the current SSD) and enter the SSD using various input elements discussed herein.

In another example, the one or more processors may use optical distance indicators (ODIs). The radiotherapy machine may be equipped with ODIs that use a projected light scale to visually display the distance from the source to the patient. This allows the system to measure and display the SSD based on the position of the treatment couch and gantry.

In another example, the one or more processors may use imaging systems integrated into the radiotherapy machine and/or the treatment room. In some embodiments, the one or more processors may use advanced imaging technologies, such as CT, X-ray, or surface mapping, to provide 3D representations of the patient's anatomy. The one or more processors can use these images to calculate the exact distance from the source to the treatment surface (e.g., skin) by referencing the machine's isocenter.

In some embodiments, the SSD may not be static and may vary depending on the gantry angle due to changes in beam orientation, patient positioning, and/or geometric transformations that occur as the gantry rotates (or is needed to be at a particular angle). These variations may introduce complexities in SSD calculations and may necessitate dynamic adjustments to ensure proper beam targeting and dose accuracy.

In some embodiments, when the gantry is positioned at a 0-degree angle (e.g., the gantry is aligned with the vertical axis), the SSD can be directly measured as the shortest distance from the radiation source to the patient's surface. In those embodiments, the measurement may rely on direct projection along the central beam axis without angular distortions. Accordingly, the SSD calculation may not require additional geometric transformations.

In other embodiments, when the gantry angle is non-zero, the effective SSD may change due to the angular displacement of the beam relative to the patient's surface. At non-zero gantry angles, the central beam may no longer follow a purely vertical path, leading to an oblique projection onto the patient's body. As a result, the SSD calculation may need to account for various factors, including trigonometric adjustments, couch positioning, beam divergence, and/or the need for automated corrections. In some embodiments, the one or more processors may calculate the SSD at the beam central axis for the setup purposes discussed herein. Alternatively, in some embodiments, trigonometric adjustments may be required because the projection of the radiation source may move relative to the patient's surface, necessitating mathematical corrections to determine the effective SSD (e.g., when trying to achieve an "average" SSD across a surface).

In some embodiments, couch positioning and rotations may further influence SSD measurements. For instance, the treatment couch may be adjusted dynamically to maintain an optimal SSD despite variations in gantry angle. If the couch remains stationary, the apparent SSD at different angles may differ from the intended treatment distance, leading to potential misalignment. Additionally, beam divergence and angular distortions may contribute to SSD variability. For instance, as the gantry rotates, the divergence of the beam may affect the projected SSD, requiring compensatory calculations or software corrections to ensure consistent dose delivery. In some embodiments, the one or more processors may use a gantry-dependent SSD calculation model, wherein a computer model identifies the gantry angle and applies corresponding transformations to compute the correct SSD in real time.

For non-zero gantry angles, the SSD may be calculated by accounting for the angular displacement of the radiation beam relative to the patient's surface. Unlike the 0-degree gantry position, where SSD is measured directly along the central beam axis, a non-zero gantry angle SSD may be calculated using various trigonometric adjustments. The calculation may involve resolving the projection of the radiation source onto the patient's surface by using the cosine of the gantry angle to adjust the direct vertical SSD measurement. Specifically, the effective SSD can be derived by considering the geometric relationship between the radiation source, the isocenter, and the patient's surface, where the SSD at a given angle is given by the baseline SSD at 0 degrees divided by the cosine of the gantry angle. In some embodiments, to improve precision, automated systems may incorporate real-time sensor data, lookup tables, or machine learning models to correct deviations and maintain an optimal SSD throughout treatment. In some embodiments, to improve precision, automated systems may incorporate real-time sensor data, lookup tables, or machine learning models to correct deviations as part of the treatment planning so that when the plan is used an optimal SSD is maintained throughout treatment.

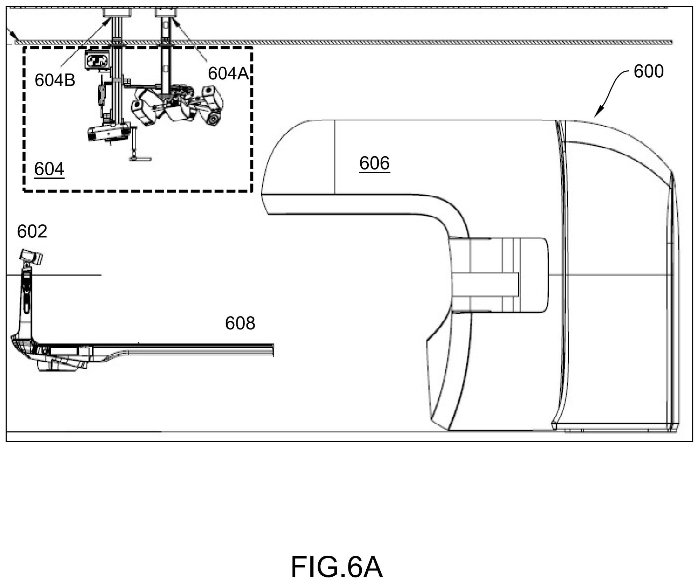

Referring now to **FIG. 6A**, the radiotherapy machine **600** may include a set of cameras. The set of cameras may be in communication with the one or more processors discussed herein, such that the one or more processors can use the data received to analyze (and sometimes display) the patient's position and alignment or SSD data. In some configurations, the one or more processors may also use the images captured by the cameras **602** and/or **604** to analyze the patient's position on the couch. For instance, the one or more processors may use the images captured by the cameras **602** and/or **604** and combine that information with a mathematical model to identify the patient's position on the couch **608.**

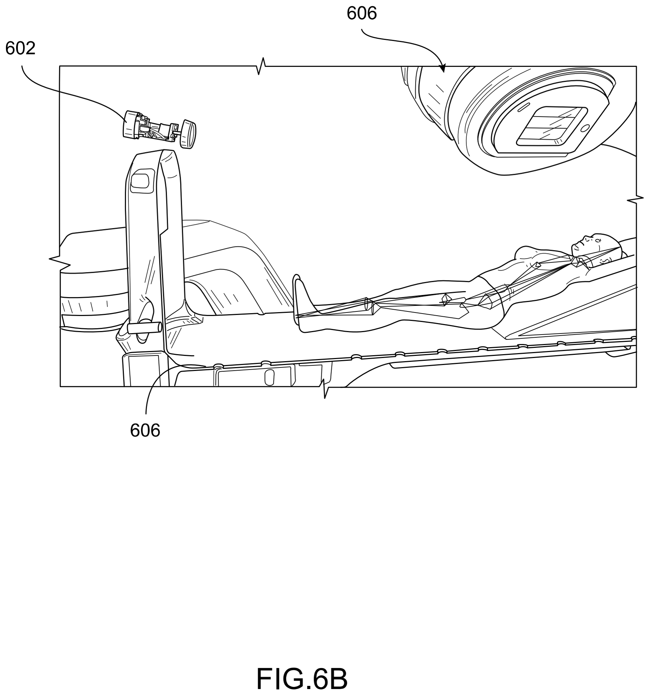

As also depicted in **FIG. 6B**, the camera **602** may be positioned at a distal end of the couch **608.** As used herein, the distal end of the couch **608** may be diametrically opposed to the proximate end of the couch **608** or to the display screen of the radiotherapy machine. The camera **602** may be disposed on a stand-alone mount or disposed on an auxiliary component of the radiotherapy machine **600**, such as the couch **608** itself. In some embodiments, the set of cameras discussed herein may be 360-degree cameras or wide-angle cameras (180-degree cameras). A 360-degree camera, as used herein, refers to a camera with a spherical field of view.

**FIGS. 6A** and **6B** illustrate a non-limiting example of cameras placed within the treatment room is illustrated. For instance, the treatment room may include a radiotherapy machine **600** (gantry **604** and couch **608**) along with the camera **602** described herein. The treatment room may include cameras **604** placed on the ceiling that can monitor various attributes described herein. For instance, the cameras **604** may be pointed towards the patient where a live feed of the patient is transmitted to the one or more processors. As a result, the one or more processors may use various methods (e.g., triangulation from multiple cameras) to identify the patient's position on the couch **608** or the gantry's position itself.

The one or more processors may adjust the cameras **604** and point the cameras **604** towards a desired location, for instance by swiveling the cameras **604** at the mounts **604A-B.** In another configuration (not shown), the cameras **604** can move along tracks in one or more directions. In yet another configuration (not shown), the cameras **604** can swivel and rotate from the posts connected to the mounts **604A-B.** In some embodiments, the cameras **604** may be disposed above the radiotherapy machine **600.** As a result, the treatment room cameras may provide a top-down view (e.g., bird's eye view) of the radiotherapy machine **600** and the patient. Alternatively, the cameras **604** may provide a perspective view (e.g., angled view) of the radiotherapy machine **600** and the patient.

In some embodiments, one or more of the cameras discussed herein may be capable of determining three-dimensional (3D) surface, shape, and/or positional information of the patient and/or the radiotherapy machine, for example by the use of time-of- flight cameras, structured light cameras, or any other 3D camera known in the art. The cameras discussed herein may be rearranged at various points within the treatment room. The cameras may be disposed to provide a vertical view of the treatment room, a horizontal view of the treatment room, or a perspective view of the treatment room.

The one or more processors may be configured to execute a triangulation protocol using the live stream of the set of cameras. In some embodiments, each camera is a time-of-flight camera (e.g., range sensing, distance measuring). In an example, the one or more processors may execute photogrammetry to extract three-dimensional measurements from the captured two-dimensional subsequent images. The scale of the subsequent images may be determined, for instance by measuring various distances of the patient (e.g., length of arms, length of legs, and/or the like) and associating the measured distances of the gantry and/or couch with an image of the patient (e.g., the subsequent images), the one or more processors may determine the current position of the patient using the distances measured and the subsequent images.

In another example, the one or more processors may use integrated sensors to calculate the SSD. The one or more processors may use sensors in communication with the radiotherapy machine that can track the relative positions of the gantry, collimator, and couch. By combining these data points, the one or more processors may calculate the SSD in real-time (or near real-time) as adjustments are made.

In another example, the one or more processors may use real-time feedback loops to calculate the SSD. For instance, as the operator moves the couch or adjusts the gantry, the one or more processors may continuously recalculate the SSD.

The combination of imaging systems (sometimes integrated into the machine and/or the treatment room), sensors, and real-time updates allows the one or more processors to precisely and efficiently determine the SSD, allowing a treatment plan to be devised that provides a required SSD and/or ensuring accuracy in radiotherapy treatments.

At step **330**, the one or more processors may reposition a couch of the radiotherapy machine in accordance with a difference between the determined SSD and a pre-defined SSD. The one or more processors may reposition a couch of the radiotherapy machine to align the determined SSD with a predefined SSD specified in the treatment plan. This functionality allows the patient to be positioned accurately for optimal radiation delivery. The one or more processors may first retrieve a pre-defined SSD from the patient's treatment plan (e.g., query a radiotherapy file) and then calculate the difference between the currently determined SSD (step **320**) and the predefined SSD. Based on this difference, the one or more processors may generate control signals to adjust the position of the couch accordingly. For instance, the one or more processors may cross-reference the actual measured distance with the planned SSD. Alternatively, in some embodiments, the one or more processors may use preset parameters to calculate the SSD.

The couch can be moved in multiple axes to achieve the correct SSD. Adjustments may include vertical movements to raise or lower the couch. Though certain embodiments discussed herein are described in terms of the SSD adjustment being vertical only, the adjustment process may involve movement of the couch along one or more axes, such as vertical (up/down), longitudinal (in/out), or lateral (left/right), to minimize the discrepancy between the determined and predefined SSD values.

The couch movement may be guided by real-time feedback from sensors, imaging systems, or laser alignment tools that monitor the patient's position relative to the radiation source. In some embodiments, the adjustment may be fully automated, with the one or more processors performing the couch repositioning without requiring operator input. Alternatively, the one or more processors may present visual cues or instructions on a display screen, enabling the operator to manually reposition the couch in accordance with the calculated difference.

By dynamically adjusting the couch position to match the predefined SSD, the one or more processors ensures precise alignment of the patient with the treatment parameters, reducing the risk of dosing errors and enhancing the overall accuracy and efficiency of the radiotherapy workflow. This capability is particularly beneficial in modalities such as electron therapy, where precise SSD alignment is critical to achieving the desired therapeutic outcomes.

The process of moving (adjusting position) the couch to achieve the desired alignment can be performed iteratively to ensure precision and consistency in radiotherapy treatments. During this iterative process, the couch is adjusted incrementally (e.g., moved vertically and/or horizontally along a calculated trajectory) based on real-time feedback from the operator and/or sensors or imaging systems that monitor the patient's position relative to the radiation source. After each adjustment, the one or more processors may re-evaluate the alignment, calculating the remaining discrepancy between the current and predefined SSD or other positional parameters. If further adjustments are needed, the one or more processors may then provide updated instructions or automatically initiate another movement, repeating this cycle until the alignment is within an acceptable or tolerable threshold (e.g., the SSD is within an acceptable threshold). This iterative approach allows for fine-tuning of the patient's positioning, accommodating subtle anatomical variations or shifts during setup, and ensures that the treatment parameters are met with high accuracy. By incorporating iterative movements, the system enhances treatment precision and minimizes the risk of alignment errors, contributing to effective and safe radiotherapy delivery.

As the couch is being adjusted, the one or more processors may optionally enable the beam eye view motion on the pendant, such that the operator can also adjust the couch as needed.

In some embodiments, SSD adjustments may be consistently applied throughout different sessions for maintaining consistency. Consistent SSD adjustments ensure that the patient is positioned correctly and that the beam path matches the configuration outlined in the treatment plan.

In some embodiments, the gantry can be moved while the couch is being adjusted to achieve optimal alignment for radiotherapy treatments. Because in electron therapy, an applicator is used, adjusting the couch may be impractical when the patient is on the couch. In manual set ups, the gantry may need to remain at the proper angle, such that the operator can visually see the SSD. However, because the methods and systems discussed herein are automated and use sensors, the gantry may be moved away for an easier patient alignment.

The one or more processors may continuously readjust the couch until the SSD is within a tolerable threshold. As used herein, the tolerable threshold may be set or defined by a medical professional or a system administrator (e.g., 1 cm).

When the SSD is within the tolerable threshold, the one or more processors may prompt the operator to confirm that the patient is aligned and the SSD is correctly achieved. For instance, the one or more processors may display a prompt on the display screen of the radiotherapy machine indicating that a verification is required. As a result, the one or more processors may receive an input indicating whether the SSD adjustment is accurately performed. For instance, the operator may use various methods and input elements to confirm that the adjustment is completed and the SSD is correct.

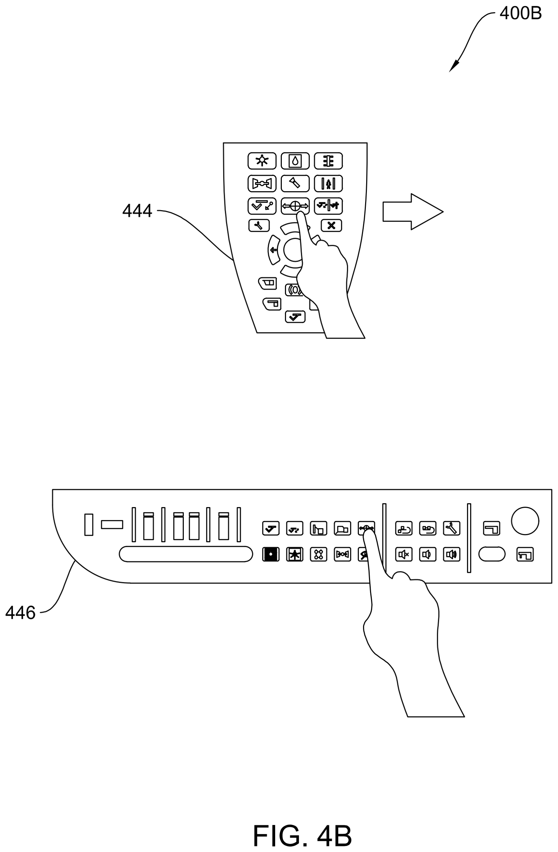

Referring now to **FIG. 4B**, the embodiment **400B** depicts two different examples of the operator providing the input to the one or more processors indicating that the SSD adjustment has been correctly performed and the correct SSD has been achieved. In the first example, the operator may use a dedicated button using the pendant (example **444**). In the second example, the operator (or another operator outside the treatment room) may use a console monitor 446 to indicate that the task has been accomplished, e.g., using a dedicated button. In some embodiments, the dedicated button may change its color when the confirming option is available (e.g., when the operator has the option to confirm correct SSD). This allows the operator to identify the dedicated button. The dedicated button may then change color after the operator has confirmed the correct SSD.

The one or more processors may be configured to adjust the treatment couch in multiple directions using a single input from the operator, thereby streamlining patient positioning and enhancing workflow efficiency during radiotherapy set up procedures. This adjustment may be executed through a dedicated button on a pendant (or any other input device) and/or via interactions with an input element on the user interface, such as using the input elements of a pendant to click an icon on the user interface. Upon receiving the instruction from the operator, the one or more processors can determine the necessary multi-directional adjustments based on pre-configured parameters, patient alignment data, and/or real-time sensor feedback. These adjustments may include vertical displacement, lateral shifts, longitudinal movements, or rotational modifications to achieve a defined SSD.

In some embodiments, the one or more processors may compute the optimal trajectory for the couch using stored patient setup data, anatomical landmarks, or imaging-based feedback. The one or more processors may actuate an engine associated with the couch to move the couch in accordance with the computed trajectory. By enabling multi-directional couch positioning through a single action, the one or more processors may reduce manual intervention, minimize setup time, and may enhance precision, ultimately improving the overall efficiency. For instance, unlike conventional methods, the operator is no longer required to use different instructions for different movements of the couch (e.g., use different actions to move the couch laterally then vertically, and then longitudinally).

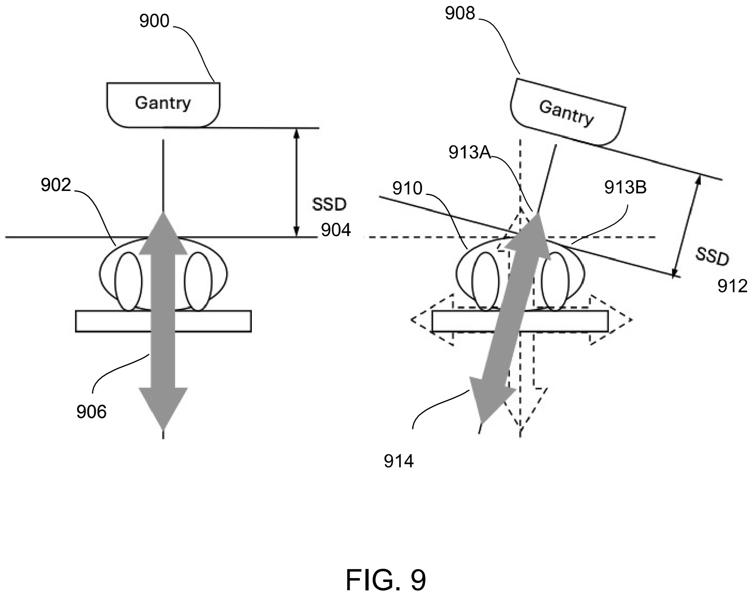

**FIG. 9** shows two examples of couch movement to achieve a defined SSD, in accordance with embodiments. **FIG. 9** illustrates two embodiments where the gantry is at a zero degree and a non-zero degree.

In the first embodiment, a gantry **900** is shown in a zero-angle orientation, such that the radiation beam is directed perpendicularly to the treatment surface of a patient **902.** In this embodiment, an SSD **904** can be calculated using the methods/systems discussed herein. The one or more processors can then adjust the couch using a single vertical movement, represented by a movement trajectory **906.**

In the second embodiment, however, the gantry **908** is positioned at an angle (e.g., angles **913A-B**)**.** Unlike the zero-angle embodiment, the beam axis is no longer aligned with a single cardinal direction, making direct vertical couch adjustments insufficient and incomplete. Instead, adjustments may need to be made along a tilted beam axis, requiring a combination of both vertical and lateral couch motions.

The gantry **908** being at an angle introduces additional complexity for the operator, as manual SSD adjustments necessitate careful coordination of multiple couch movement directions to maintain the correct alignment along the angled beam axis. To simplify this process, the one or more processors may use a logic-based approach that enables couch motion along the tilted beam axis using a single input element or instructions by the operator. For instance, the couch can be iteratively moved in multiple directions until the SSD is within a tolerable threshold.

The one or more processors may calculate a trajectory **914** for the couch **910** that incudes movements in multiple directions (e.g., vertical, lateral, and/or longitudinal) to achieve an SSD in accordance with the current angle of the gantry **908.** Instead of requiring the operator to manually coordinate multiple directional adjustments, the one or more processors may allow SSD modifications to be executed through a single user input. Accordingly, when the one or more processors receive an indication that the operator has instructed the couch to be moved, the one or more processors may move the couch in accordance with the trajectory **914** to achieve the SSD along the titled beam axis. In some embodiments, the one or more processors may compensate for the gantry's angular displacement, automatically adjusting the lateral position as needed until the defined SSD is achieved. This approach reduces the cognitive load on the operator, minimizes setup time, and enhances the accuracy and efficiency of SSD adjustments during radiotherapy treatments.

In some embodiments, the operator may be prompted to confirm patient alignment and correct SSD by aligning the patient with pre-generated marks or reference points. Reference marking in radiotherapy may involve creating physical or visual markers (sometimes referred to as reference points) on the patient or their immobilization devices (accessories) to establish precise alignment points, ensuring consistent positioning. These markers may act as alignment guides, ensuring that the patient's position on the treatment couch matches the planned position from the imaging and treatment planning stages.

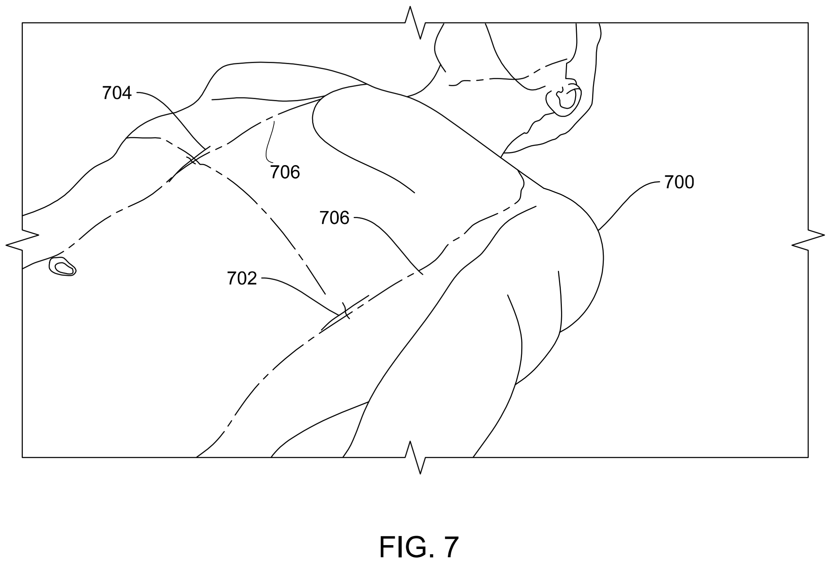

In a non-limiting example, the process begins during the initial imaging session, such as a CT scan, where reference marks are applied to the patient or their immobilization device. For instance, as depicted in **FIG. 7**, markers **702** and **704** may be placed on the patient **700**, e.g., using tape or other markers. These markers may be subsequently used in the treatment room to correlate with the lasers emitted on the patient **700** (laser beam **706**), ensuring that any shifts or adjustments made during imaging are quantitatively translated into the treatment setup.

During the alignment process, the operator may use laser guidance systems integrated into the treatment room to align the patient's position with the reference marks previously established during the imaging session (markers **702** and **704**)**.** The lasers may present a visual aid that ensures the patient's position on the treatment couch is correctly aligned with the radiotherapy machine's isocenter, thereby maintaining the precision necessary for effective treatment delivery.

Once the laser output (or any other indicator emitted onto the patient, such as any light source) is aligned with the markers, the operator confirms that the emitted laser beam **506** coincides with the reference marks using a button on the pendant or an interactive element on the GUI. This input signals the one or more processors to record that the patient is aligned and ready for subsequent workflow stages and/or tasks. By incorporating this alignment and confirmation process, the disclosed methods and systems ensure consistency, accuracy, and efficiency in patient setup, thereby reducing the risk of errors and enhancing overall treatment quality. This aligning paradigm provides a reliable and repeatable method for maintaining alignment consistency, which is crucial for successful radiotherapy outcomes.

The positions of the reference markers relative to the lasers may provide a measurable framework for making adjustments to the patient's positioning. Operators may use this information to fine-tune the patient's placement on the treatment couch, ensuring alignment with the machine's isocenter.

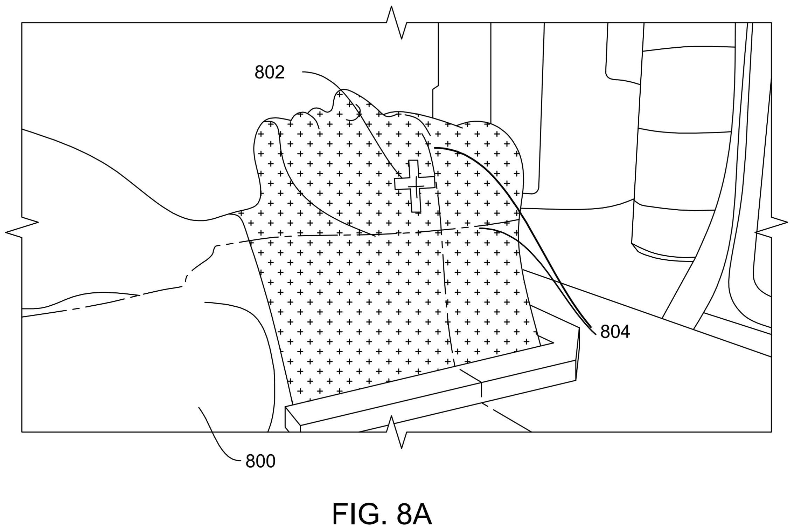

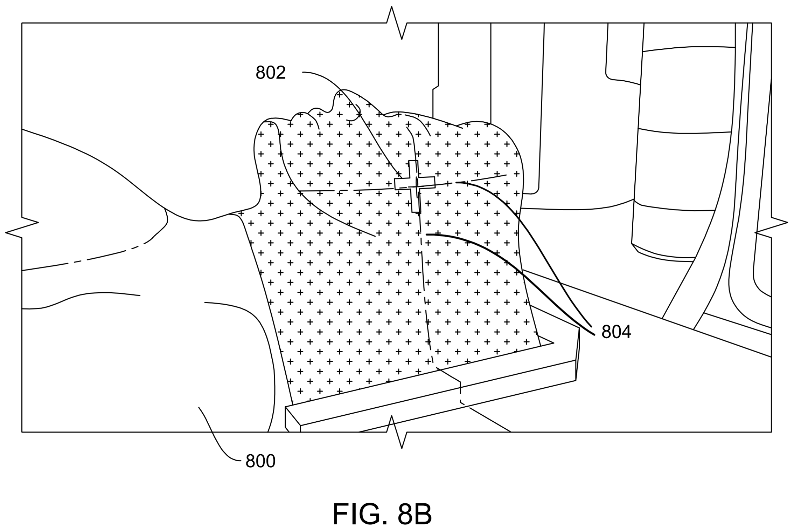

In some embodiments, the markers may be applied to an accessory, such as an immobilization device/mask. This process may be particularly critical for treatment areas such as the head and neck, where immobilization masks are frequently used. For example, as depicted in **FIG. 8A**, an indicator **802** may be tape or ink used to create visible markers on a mask worn by the patient **800.** The indicator **802** may be designed to align with the laser beam **804** emitted onto the patient **800** in the imaging room. Once the indicator **802** and the laser beam **804** are aligned (**FIG. 8B**), the operator may confirm the alignment and the one or more processors may move to the subsequent stage or step of the method **300.**

Alternatively, the one or more processors may retrieve data from an external source, such as the radiotherapy machine itself and/or other sensors or cameras within the treatment room. In this way, the operator is no longer required to provide a confirmation of correct SSD or alignment.

Referring back to **FIG. 3**, at step **340**, the one or more processors may present, on the display screen, a visual indicator indicating that the correct SSD has been achieved. The visual indicator may provide a visual confirmation to the operator that the radiotherapy machine, including components such as the gantry, couch, and beam delivery system, is correctly positioned according to the parameters defined in the treatment plan, such that the correct SSD is achieved.

The visual indicator may take various forms, such as a dedicated icon or indicator that indicates proper SSD, a checkmark, a flashing signal, or an animation, to signify successful alignment. For example, a green checkmark icon may appear on the display screen once the correct SSD is confirmed, signaling that the operator can move to the next step. In another example, a portion of the GUI may change color (e.g., green is shown on top and bottom or sides of the center portion). Moreover, in some embodiments, a color-coded icon could indicate alignment status, with green signifying successful alignment and red signaling an error or misalignment that requires operator intervention.

In some embodiments, an animated checkmark may appear on the GUI, completing its animation (e.g., status bar) when alignment is verified, providing a visually engaging and clear confirmation for the operator. For instance, a progress bar representing the alignment process could fill completely and/or turn green when the alignment is successfully achieved, offering a clear visual cue.

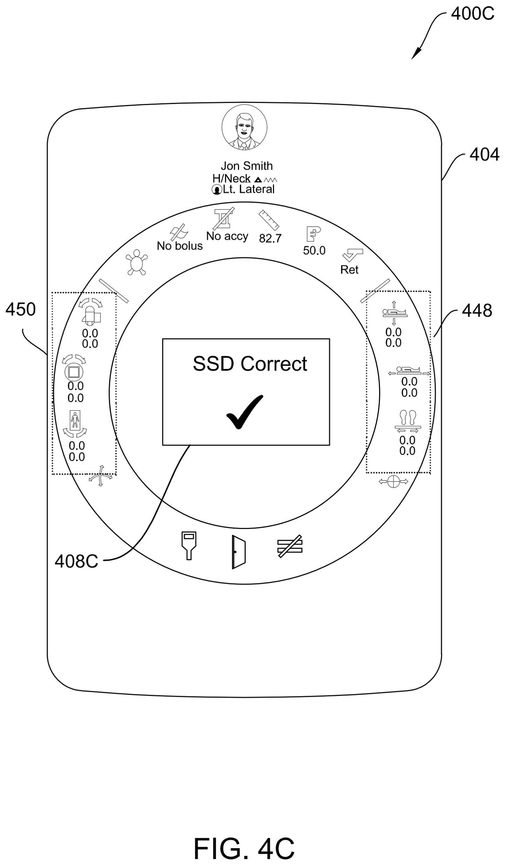

Referring to **FIG. 4C**, the one or more processors may revise the center portion **408C** to display a message indicating that the alignment has been completed. The message may also include a checkmark (or any other dedicate visual marking in any defined color) indicating that the alignment stage has been successfully completed.

After receiving the input that the correct SSD has been achieved, the one or more processors may configure the machine parameters to a default position or default configuration. These default attributes may establish a consistent baseline for operational parameters, ensuring the machine is initialized in alignment with standard practices or specific treatment protocols. Key machine parameters (e.g., gantry and couch position) may be set to predefined values (e.g., 0). As depicted, the icons **448** and **450** reflect zeros indicating that the couch is at a default position.

Setting the machine parameters to a default position allows for easier readjustment and/or additional adjustments because the subsequent adjustments can be conveyed in terms of the default position of the machine and not the original position of the machine. In some embodiments, after achieving the correct SSD, the couch and/or the patient may be moved for other alignment purposes. As a result, the couch and/or the patient might need to be readjusted. However, instead of providing adjustment attributes based on the original position of the couch (or other parts of the radiotherapy machine), the one or more processors may display the adjustment values based on the new baseline (the default position).

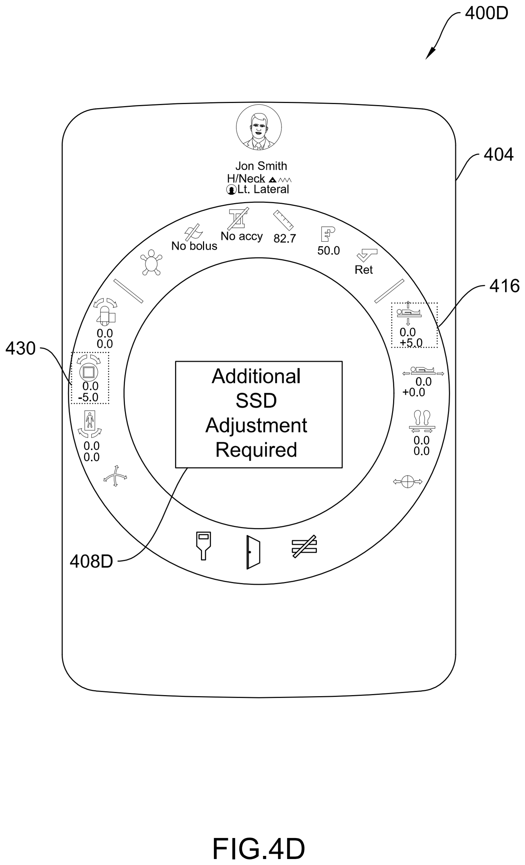

Referring now to **FIG. 4D**, embodiment **400D** depicts an example where the machine has been zeroed before additional changes are required. As a result, the center portion **408D** is dynamically revised to indicate that additional SSD adjustments are required. Moreover, values for icons **416** (vertical movement of the couch) and **430** (rotation of the gantry) are revised to reflect couch adjustment and gantry angles relative to the zeroed or default position. As depicted, the adjustment values are with respect to the zeroed position (e.g., default configuration) and not the original position with which the values depicted in **FIG. 4A** were calculated. This approach allows operators to visualize and track all subsequent adjustments clearly, maintaining an accurate and consistent alignment framework.

In some embodiments, one or more settings and attributes, as configured using the methods discussed herein, can be saved to ensure consistency and efficiency across multiple treatment sessions. By storing the configured attributes, such as couch position (e.g., vertical), the radiotherapy machine can be initialized with the same configuration at the start of each subsequent session. For instance, SSD values and couch position can be saved such that the one or more processors can adjust the couch before the next treatment of the patient. This capability eliminates the need for repetitive setup processes, reducing operator workload and minimizing the potential for errors during reconfiguration. Additionally, saving these settings ensures that treatment parameters remain aligned with the patient's treatment plan, enabling seamless continuity across sessions. This functionality is particularly beneficial in cases where precise and repeatable configurations are critical for achieving optimal treatment outcomes, such as in multi-fraction radiotherapy regimens. By allowing operators to retrieve and apply saved configurations, the system enhances workflow efficiency and supports high-precision treatment delivery.

### Example clauses

Further aspects of these teachings are provided by the subject matter of the following clauses.

Clause 1. A computer-readable medium comprising a set of instructions, that when executed, cause at least one processor to: present, on a display screen located on a gantry of a radiotherapy machine configured to implement radiotherapy treatment of a patient, a set of icons corresponding to a set of instructions associated with adjusting at least one attribute of the radiotherapy machine before the radiotherapy treatment starts; calculate a source to skin distance (SSD) for the patient; adjust a couch of the radiotherapy machine, such that the SSD is within a tolerable threshold of a defined SSD; and present, on the display screen, a visual indicator indicating that a correct SSD has been achieved.

Clause 2. The computer-readable medium of clause 1, wherein the instructions further cause the at least one processor to: receive an input confirming that the correct SSD is achieved.

Clause 3. The computer-readable medium of clauses 1-2, wherein the input is received via an interaction with the display screen or via a pendant of the radiotherapy machine.

Clause 4. The computer-readable medium of clauses 1-3, wherein the input is received after aligning a marking on a patient with an indicator emitted on the patient.

Clause 5. The computer-readable medium of clauses 1-4, wherein the visual indicator is an additional icon displayed on the display screen or corresponds to a change of a visual attribute for at least one icon within the set of icons.

Clause 6. The computer-readable medium of clauses 1-5, wherein the instructions further cause the at least one processor to: configure the at least one attribute of the radiotherapy machine as a default attribute.

Clause 7. The computer-readable medium of clauses 1-6, wherein the instructions further cause the at least one processor to: identify an additional adjustment to the at least one attribute; and present the additional adjustment in terms of a default configuration and not an original radiotherapy configuration.

Clause 8. A computer system comprising: a radiotherapy machine having a display screen; and at least one processor in communication with the display screen, the processor configured to: present, on the display screen located on a gantry of the radiotherapy machine configured to implement radiotherapy treatment of a patient, a set of icons corresponding to a set of instructions associated with adjusting at least one attribute of the radiotherapy machine before the radiotherapy treatment starts; calculate a source to skin distance (SSD) for the patient; adjust a couch of the radiotherapy machine, such that the SSD is within a tolerable threshold of a defined SSD; and present, on the display screen, a visual indicator indicating that a correct SSD has been achieved.

Clause 9. The computer system of clause 8, wherein the at least one processor is further configured to: receive an input confirming that the correct SSD is achieved.

Clause 10. The computer system of clauses 8-9, wherein the input is received via an interaction with the display screen or via a pendant of the radiotherapy machine.

Clause 11. The computer system of clauses 8-10, wherein the input is received after aligning a marking on a patient with an indicator emitted on the patient.

Clause 12. The computer system of clauses 8-11, wherein the visual indicator is an additional icon displayed on the display screen or corresponds to a change of a visual attribute for at least one icon within the set of icons.

Clause 13. The computer system of clauses 8-12, wherein the at least one processor is further configured to configure the at least one attribute of the radiotherapy machine as a default attribute.

Clause 14. A method comprising: presenting, by at least one processor on a display screen located on a gantry of a radiotherapy machine configured to implement radiotherapy treatment of a patient, a set of icons corresponding to a set of instructions associated with adjusting at least one attribute of the radiotherapy machine before the radiotherapy treatment starts; calculating, by the at least one processor, a source to skin distance (SSD) for the patient; adjusting, by the at least one processor, a couch of the radiotherapy machine, such that the SSD is within a tolerable threshold of a defined SSD; and presenting, by the at least one processor on the display screen, a visual indicator indicating that a correct SSD has been achieved.

Clause 15. The method of clause 14, further comprising: receiving, by the at least one processor, an input confirming that the correct SSD is achieved.

Clause 16. The method of clauses 14-15, wherein the input is received via an interaction with the display screen or via a pendant of the radiotherapy machine.

Clause 17. The method of clauses 14-16, wherein the input is received after aligning a marking on a patient with an indicator emitted on the patient.

Clause 18. The method of clauses 14-17, wherein the visual indicator is an additional icon displayed on the display screen or corresponds to a change of a visual attribute for at least one icon within the set of icons.

Clause 19. The method of clauses 14-18, further comprising: configuring, by the at least one processor, the at least one attribute of the radiotherapy machine as a default attribute.

Clause 20. The method of clauses 14-19, further comprising: identifying, by the at least one processor, an additional adjustment to the at least one attribute; and presenting, by the at least one processor, the additional adjustment in terms of a default configuration and not an original radiotherapy configuration.

Clause 21: A computer program comprising instructions which, when the program is executed by a computer, cause the computer to carry out the method of any of clauses 14-20.

Clause 22: A computer-readable medium comprising a set of instructions, that when executed by one or more processors, cause the one or more processors to carry out the method of any of clauses 14-20.

The presentation of information on the display screen, the user interaction with the display screen and/or the GUI, may have the technical effect of assisting the user in performing a radiotherapy setup workflow by means of a continued and/or guided process of human-machine interaction. The systems discussed herein may be configured to assist the user in performing a radiotherapy workflow and/or a radiotherapy setup workflow, by means of a continued and/or guided process of human-machine interaction.

In this disclosure, the term "radiotherapy treatment" may refer to radiotherapy treatment and/or radiotherapy treatment setup. The method steps may refer to treatment setup, while the systems may be configured to perform treatment and/or treatment setup.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the invention. Thus, the operation and behavior of the systems and methods were described without reference to the specific software code being understood that software and control hardware can be designed to implement the systems and methods based on the description herein.

When implemented in software, the functions may be stored therein as one or more instructions or code on a computer-readable, non-transitory medium or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the present invention. Various modifications to these embodiments will be readily apparent to those skilled in the art, and the generic principles defined herein may be applied to other embodiments without departing from the spirit or scope of the invention. Thus, the present invention is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope and spirit being indicated by the following claims.

## Claims

1. A computer-readable medium comprising a set of instructions, that when executed, cause at least one processor to:
present, on a display screen coupled to a radiotherapy machine configured to implement radiotherapy treatment of a patient, a set of icons corresponding to a set of instructions associated with adjusting at least one attribute of the radiotherapy machine before the radiotherapy treatment starts;
calculate a source to skin distance, SSD, for the patient;
adjust a couch and/or a source of the radiotherapy machine, such that the SSD is within a tolerable threshold of a defined SSD; and
present, on the display screen, a visual indicator indicating that a correct SSD has been achieved.

2. The computer-readable medium of claim 1, wherein the instructions further cause the at least one processor to:
receive an input confirming that the correct SSD is achieved.

3. The computer-readable medium of claim 1 or 2, wherein the instructions further cause the at least one processor to:
configure the at least one attribute of the radiotherapy machine as a default attribute.

4. The computer-readable medium of claim 3, wherein the instructions further cause the at least one processor to:
identify an additional adjustment to the at least one attribute; and
present the additional adjustment in terms of a default configuration and not an original radiotherapy configuration.

5. A computer system comprising:
a radiotherapy machine having a display screen; and
at least one processor in communication with the display screen, the processor configured to:
present, on the display screen coupled to the radiotherapy machine configured to implement radiotherapy treatment of a patient, a set of icons corresponding to a set of instructions associated with adjusting at least one attribute of the radiotherapy machine before the radiotherapy treatment starts;
calculate a source to skin distance, SSD, for the patient;
adjust a couch and/or a source of the radiotherapy machine, such that the SSD is within a tolerable threshold of a defined SSD; and
present, on the display screen, a visual indicator indicating that a correct SSD has been achieved.

6. The computer system of claim 5, wherein the at least one processor is further configured to:
receive an input confirming that the correct SSD is achieved.

7. The computer system of claim 5 or 6, wherein the at least one processor is further configured to configure the at least one attribute of the radiotherapy machine as a default attribute.

8. A treatment setup method comprising:
presenting, by at least one processor on a display screen coupled to a radiotherapy machine configured to implement radiotherapy treatment of a patient, a set of icons corresponding to a set of instructions associated with adjusting at least one attribute of the radiotherapy machine before the radiotherapy treatment starts;
calculating, by the at least one processor, a source to skin distance, SSD, for the patient;
adjusting, by the at least one processor, a couch and/or a source of the radiotherapy machine, such that the SSD is within a tolerable threshold of a defined SSD; and
presenting, by the at least one processor on the display screen, a visual indicator indicating that a correct SSD has been achieved.

9. The method of claim 8, further comprising:
receiving, by the at least one processor, an input confirming that the correct SSD is achieved.

10. The computer-readable medium of claim of claim 2, the computer system of claim 6 or the method of claim 9, wherein the input is received via an interaction with the display screen or via a pendant of the radiotherapy machine.

11. The computer-readable medium of claim of claim 2 or 10, the computer system of claim 6 or 10, or the method of claim 9 or 10, wherein the input is received after aligning a marking on a patient with an indicator emitted on the patient.

12. The computer-readable medium, computer system or method of claim 11, wherein the visual indicator is an additional icon displayed on the display screen or corresponds to a change of a visual attribute for at least one icon within the set of icons.

13. The method of any one of claims 8 to 12, further comprising:
configuring, by the at least one processor, the at least one attribute of the radiotherapy machine as a default attribute.

14. The method of claim 13, further comprising:
identifying, by the at least one processor, an additional adjustment to the at least one attribute; and
presenting, by the at least one processor, the additional adjustment in terms of a default configuration and not an original radiotherapy configuration.

15. The computer-readable medium, computer system or method of any one of the preceding claims, wherein adjusting the couch of the radiotherapy machine, such that the SSD is within a tolerable threshold of the defined SSD, includes iteratively calculating a difference between the SSD and the defined SSD and presenting visual cues or instructions on the display screen indicative of the difference between the SSD and the defined SSD, enabling an operator to manually reposition the couch in accordance with the calculated difference.
